# EUROPEAN PATENT APPLICATION

(11) **EP 4 501 328 A1**
(43) Date of publication of application: **05.02.2025**
(21) Application number: 23780975.1
(22) Date of filing: 30.03.2023
(51) Int. Cl.: A61K 31/53, A61P 35/00, C12Q 1/6869, C12Q 1/6886, G01N 33/574

(54) **BIOMARKER FOR TREATMENT OF SOLID CANCER BY IMIDAZO[4,5-B]PYRIDINE DERIVATIVE**

(30) Priority: 31.03.2022 JP 2022060240
(71) Applicant: Chordia Therapeutics Inc., Fujisawa-shi, Kanagawa 251-0012 (JP); National Cancer Center, Tokyo 104-0045 (JP)
(72) Inventor: TOZAKI Hirokazu, Fujisawa-shi, Kanagawa 251-0012 (JP); MORISHITA Daisuke, Fujisawa-shi, Kanagawa 251-0012 (JP); MIZUTANI Akio, Fujisawa-shi, Kanagawa 251-0012 (JP); SATOH Yoshihiko, Fujisawa-shi, Kanagawa 251-0012 (JP); HIKAMI Koki, Fujisawa-shi, Kanagawa 251-0012 (JP); MANO Hiroyuki, Tokyo 104-0045 (JP); KOHSAKA Shinji, Tokyo 104-0045 (JP); HAMADA Akinobu, Tokyo 104-0045 (JP); YAGISHITA Shigehiro, Tokyo 104-0045 (JP); IWAI Kenichi, Fujisawa-shi, Kanagawa 251-0012 (JP); YAGUCHI Masahiro, Fujisawa-shi, Kanagawa 251-0012 (JP); KUMAZAWA Kazuho, Fujisawa-shi, Kanagawa 251-0012 (JP); NOMURA Toshiyuki, Fujisawa-shi, Kanagawa 251-0012 (JP)
(74) Representative: Weickmann & Weickmann PartmbB
(86) International application number: PCT/JP2023/013361
(87) International publication number: WO 2023/190967

(57) **Abstract**

Provided is a means to predict the anticancer therapeutic efficacy of 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine or a derivative thereof in solid cancer and thereby specifying a subject who will be the most benefitted from treatment with the compound and treatment with the compound in combination with another drug and/or another therapy. As a biomarker for predicting therapeutic response to the compound, a genetic character predicted to impart sensitivity to CLK inhibition is used.

## Description

### Technical Field

The present invention relates to use of a MYC gene and other cancer-related genes as biomarkers for predicting therapeutic response to an imidazo[4,5-b]pyridine derivative in a subject with solid cancer, methods for diagnosing and treating solid cancer with such a biomarker, and a pharmaceutical composition characterized by such a biomarker for treating solid cancer.

### Background Art

Imidazo[4,5-b]pyridine derivatives, in particular, 1-((5-(1R)-1-fluoroethyl)-1,3,4-oxadiazol-yl)methyl)-6-(4-methoxypyrrolo[2,1-f] [1,2,4]triazin-5-yl)-2-methyl-1H-imidazo[4,5-b]pyridine (hereinafter, referred to as compound A) represented by the following formula (I): 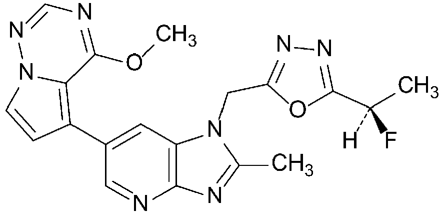 or a derivative thereof is an inhibitor for CLK (Cdc2-like kinase) that primarily induces skipped-exon-type splicing alteration to exhibit potent antitumor activity. Patent Literature 1 demonstrates that a CLK inhibitor in which compound A or a derivative thereof is included, is useful for preventing and/or treating CLK-related diseases including cancers, and exerts antitumor effect in various in vivo cancer models including those of ovarian cancer, breast cancer, gastric cancer, lung cancer, large intestine carcinoma, blood cancer, and prostate cancer.

### Document List

### Patent Literature

Patent Literature 1: International Publication No. WO 2017/188374

### Summary of Invention

### Technical Problem

An object of the present invention is to predict the anticancer therapeutic efficacy of compound A or a derivative thereof in solid cancer and thereby specify a subject who will be the most benefitted from treatment with compound A or a derivative thereof and treatment with compound A or a derivative thereof in combination with another drug and/or another therapy.

### Solution to Problem

The present inventors have found a genetic character that exhibits particularly high responsivity to compound A or a derivative thereof and is predicted to impart sensitivity to CLK inhibition in solid cancer, and diligently studied on the basis of the finding, eventually completing the present invention. Specifically, the present invention is as follows.
[1] A method for predicting therapeutic response to 1-((5-(1R)-1-fluoroethyl)-1,3,4-oxadiazol-yl)methyl)-6-(4-methoxypyrrolo[2,1-f] [1,2,4]triazin-5-yl)-2-methyl-1H-imidazo[4,5-b]pyridine represented by the following formula (I): 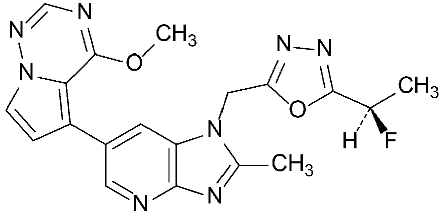 or a derivative thereof in a subject with solid cancer, the method comprising: (1) detecting a genetic character predicted to impart sensitivity to CLK inhibition in a cancer-tissue-derived sample from the subject; and (2) determining an expectation of response to the compound represented by the formula (I) or a derivative thereof in the subject with reference to the genetic character.
[2] The method according to item [1], wherein the genetic character is one or more selected from the group consisting of:
   (a) MYC gene amplification;
   (b) MYC gene mutation;
   (c) MYC gene chromosomal transposition;
   (d) MYC mRNA level elevation;
   (e) MYC protein level elevation;
   (f) EGFR gene mutation;
   (g) U2AF1 gene mutation;
   (h) POLE gene reduction;
   (i) CDK12 gene reduction;
   (j) mutation of a gene selected from one or more of ATRX, ATR, GNAQ, and HIST1H3B; and
   (k) MDM4 exon skip alteration.
[3] The method according to item [2], comprising:
   (1) detecting MYC gene amplification, MYC gene mutation, MYC gene chromosomal transposition, MYC mRNA level elevation, and/or MYC protein level elevation in the cancer-tissue-derived sample from the subject; and
   (2) determining that the subject has particularly high responsivity to the compound represented by the formula (I) or a derivative thereof if a MYC gene copy number is increased as compared with a reference MYC gene copy number has been found, a MYC gene sequence is altered as compared with areference MYC gene sequence, a MYC gene chromosomal position or site is translocated as compared with a reference MYC gene chromosomal position or site, a MYC mRNA level is elevated as compared with a reference MYC mRNA level, and/or a MYC protein level is elevated as compared with a reference MYC protein level.
[4] The method according to item [3], wherein the MYC gene is selected from the group consisting of MYCC, MYCN, and MYCL.
[5] The method according to item [3], wherein the MYC gene copy number is shown to be approximately 1.5 times or more increased as compared with the reference MYC gene copy number, or the MYC gene sequence is shown to have MYCC Q37del, P59L, S146L, MYCN R45Rfs*86, or E445K as compared with the reference.
[6] The method according to item [3], wherein the MYC gene amplification or MYC gene mutation is detected by array comparative genomic hybridization (aCGH), single-nucleotide polymorphism (SNP) arrays, copy number variation (CNV) sequencing, next-generation sequencing (NGS), or multiplex ligation-dependent probe amplification (MLPA).
[7] The method according to item [3], wherein transposition of the MYC gene chromosomal position or site is detected by in situ hybridization (ISH), fluorescence in situ hybridization (FISH), next-generation sequencing (NGS), or comparative genomic hybridization (CGH).
[8] The method according to item [3], wherein the MYC mRNA level elevation is detected by in situ hybridization (ISH), fluorescence in situ hybridization (FISH), next-generation sequencing (NGS), or real-time PCR or digital PCR analysis.
[9] The method according to item [3], wherein the MYC protein level elevation is detected by immunohistochemical staining (IHC), flow cytometry (FACS), enzyme-linked immunosorbent assay (ELISA), proteinarray (Proximity Extension Assay, PEA method), or Western blotting (WB).
[10] The method according to item [2], comprising:
   (1) detecting EGFR gene mutation, U2AF1 gene mutation, POLE gene reduction, CDK12 gene reduction, mutation of a gene selected from one or more of ATRX, ATR, GNAQ, and HIST1H3B, and/or MDM4 exon skip alteration in the cancer-tissue-derived sample from the subject; and
   (2) determining that the subject has particularly high responsivity to the compound represented by the formula (I) or a derivative thereof if a sequence of a gene selected from one or more of an EGFR gene, a U2AF1 gene, ATRX, ATR, GNAQ, and HIST1H3B is altered as compared with a reference gene sequence for the corresponding gene, and/or an MDM4 mRNA sequence is altered as compared with reference MDM4 mRNA sequence, and/or a POLE gene copy number or CDK12 gene copy number is decreased as compared with a reference copy number for the corresponding gene.
[11] The method according to item [10], wherein the U2AF1 gene mutation is S34F mutation or S34Y mutation.
[12] The method according to item [10] or [11], wherein the EGFR gene mutation, the U2AF1 gene mutation, the POLE gene reduction, the CDK12 gene reduction, the mutation of a gene selected from one or more of ATRX, ATR, GNAQ, and HIST1H3B, and/or the MDM4 exon skip alteration are/is detected by array comparative genomic hybridization (aCGH), single-nucleotide polymorphism (SNP) arrays, copy number variation (CNV) sequencing, multiplex ligation-dependent probe amplification (MLPA), or RNA sequencing.
[13] The method according to any one of items [1] to [12], wherein the cancer-tissue-derived sample is a surgical sample, biopsy sample, pleural fluid sample, or ascitic fluid sample obtained from the subject, and, for example, is a sample collected from one or more of tissues of a lung, blood, a breast, a gastrointestinal tract (a stomach, a colon, a rectum), an ovary, a pancreas, a liver, a bladder, a uterine cervix, a nerve, a uterus, a salivary gland, a kidney, a prostate, a thyroid, or muscle.
[14] The method according to any one of items [1] to [12], wherein the subject is a human subject.
[15] The method according to any one of items [1] to [12], wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.
[16] The method according to any one of items [3] to [9], wherein the MYC gene is MYCC, and the cancer is selected from ovarian cancer, breast cancer, gastric cancer, lung cancer, large intestine carcinoma, and/or prostate cancer.
[17] The method according to [16], wherein the MYC gene is MYCC, and the cancer is ovarian cancer.
[18] The method according to any one of items [3] to [9], wherein the MYC gene is MYCN, and the cancer is selected from ovarian cancer, breast cancer, gastric cancer, lung cancer, large intestine carcinoma, prostate cancer, neuroblastoma, small-cell lung cancer, alveolar rhabdomyosarcoma, medulloblastoma, glioblastoma multiforme, and/or retinoblastoma.
[19] The method according to [18], wherein the MYC gene is MYCN, and the cancer is neuroblastoma.
[20] The method according to any one of items [3] to [9], wherein the MYC gene is MYCL, and the cancer is selected from ovarian cancer, breast cancer, gastric cancer, lung cancer, large intestine carcinoma, and/or prostate cancer.
[21] A biomarker selected from the following (a) to (k):
   (a) MYC gene amplification;
   (b) MYC gene mutation;
   (c) MYC gene chromosomal transposition;
   (d) MYC mRNA level elevation;
   (e) MYC protein level elevation;
   (f) EGFR gene mutation;
   (g) U2AF1 gene mutation;
   (h) POLE gene reduction;
   (i) CDK12 gene reduction;
   (j) mutation of a gene selected from one or more of ATRX, ATR, GNAQ, and HIST1H3B; and
   (k) MDM4 exon skip alteration
   for predicting therapeutic response to a therapy with 1-((5-(1R)-1-fluoroethyl)-1,3,4-oxadiazol-yl)methyl)-6-(4-methoxypyrrolo[2,1-f] [1,2,4]triazin-5-yl)-2-methyl-1H-imidazo[4,5-b]pyridine represented by the following formula (I): 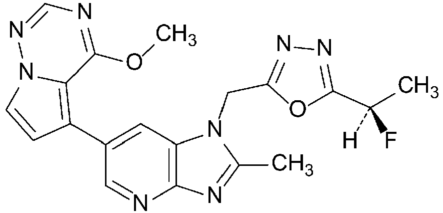 or a derivative thereof in a subject with solid cancer.
[22] The biomarker according to item [21], wherein the MYC gene is selected from the group consisting of MYCC, MYCN, and MYCL.
[23] The biomarker according to item [21], wherein the mutation of the U2AF1 gene is S34F mutation or S34Y mutation.
[24] The biomarker according to any one of items [21] to [23], wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.
[25] The biomarker according to item [21], wherein the MYC gene is MYCC, and the cancer is selected from ovarian cancer, breast cancer, gastric cancer, lung cancer, large intestine carcinoma, and prostate cancer.
[26] The biomarker according to item [25], wherein the MYC gene is MYCC, and the cancer is ovarian cancer.
[27] The biomarker according to item [21], wherein the MYC gene is MYCN, and the cancer is selected from ovarian cancer, breast cancer, gastric cancer, lung cancer, large intestine carcinoma, prostate cancer, neuroblastoma, small-cell lung cancer, alveolar rhabdomyosarcoma, medulloblastoma, glioblastoma multiforme, and retinoblastoma.
[28] The biomarker according to item [27], wherein the MYC gene is MYCN, and the cancer is neuroblastoma.
[29] The biomarker according to item [21], wherein the MYC gene is MYCL, and the cancer is selected from ovarian cancer, breast cancer, gastric cancer, lung cancer, large intestine carcinoma, and prostate cancer.
[30] A pharmaceutical composition for treating solid cancer having a genetic character predicted to impart sensitivity to CLK inhibition, the pharmaceutical composition comprising 1-((5-(1R)-1-fluoroethyl)-1,3,4-oxadiazol-yl)methyl)-6-(4-methoxypyrrolo[2,1-f] [1,2,4]triazin-5-yl)-2-methyl-1H-imidazo[4,5-b]pyridine represented by the following formula (I): 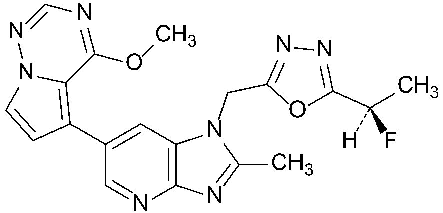 or a derivative thereof as an active ingredient, wherein
   the genetic character is one or more selected from the following (a) to (k):
   (a) MYC gene amplification;
   (b) MYC gene mutation;
   (c) MYC gene chromosomal transposition;
   (d) MYC mRNA level elevation;
   (e) MYC protein level elevation;
   (f) EGFR gene mutation;
   (g) U2AF1 gene mutation;
   (h) POLE gene reduction;
   (i) CDK12 gene reduction;
   (j) mutation of a gene selected from one or more of ATRX, ATR, GNAQ, and HIST1H3B; and
   (k) MDM4 exon skip alteration.
[31] The pharmaceutical composition according to item [30], wherein the MYC gene is selected from the group consisting of MYCC, MYCN, and MYCL.
[32] The pharmaceutical composition according to item [30], wherein the U2AF1 gene mutation is S34F mutation or S34Y mutation.
[33] The pharmaceutical composition according to any one of items [30] to [32], wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.
[34] The pharmaceutical composition according to item [30], wherein the MYC gene is MYCC, and the cancer is selected from ovarian cancer, breast cancer, gastric cancer, lung cancer, large intestine carcinoma, and prostate cancer.
[35] The pharmaceutical composition according to item [34], wherein the MYC gene is MYCC, and the cancer is ovarian cancer.
[36] The pharmaceutical composition according to item [30], wherein the MYC gene is MYCN, and the cancer is selected from ovarian cancer, breast cancer, gastric cancer, lung cancer, large intestine carcinoma, neuroblastoma, small-cell lung cancer, alveolar rhabdomyosarcoma, medulloblastoma, glioblastoma multiforme, and retinoblastoma.
[37] The pharmaceutical composition according to item [36], wherein the MYC gene is MYCN, and the cancer is neuroblastoma.
[38] The pharmaceutical composition according to item [30], wherein the MYC gene is MYCL, and the cancer is selected from ovarian cancer, breast cancer, gastric cancer, lung cancer, large intestine carcinoma, and prostate cancer.

### Effects of Invention

The present invention provides a biomarker for predicting the therapeutic efficacy of compound A or a derivative thereof in a subject with solid cancer. This enables identification of a subject who can receive higher anticancer effect from a therapy with compound A or a derivative thereof.

### Brief Description of Drawings

[Fig. 1] A diagram showing sensitivity evaluation with compound A for NIH3T3 cell lines with different cancer-related genes introduced therein.
[Fig. 2] A graph showing sensitivity evaluation with compound A for NIH3T3 cell lines having a genetic character in MYCC or MYCN.
[Fig. 3] A graph showing sensitivity evaluation with compound A for cell lines having a genetic character in MYCC, MYCN, or EGFR.
[Fig. 4] A graph showing sensitivity evaluation with compound A for cell lines having MYCC gene amplification.

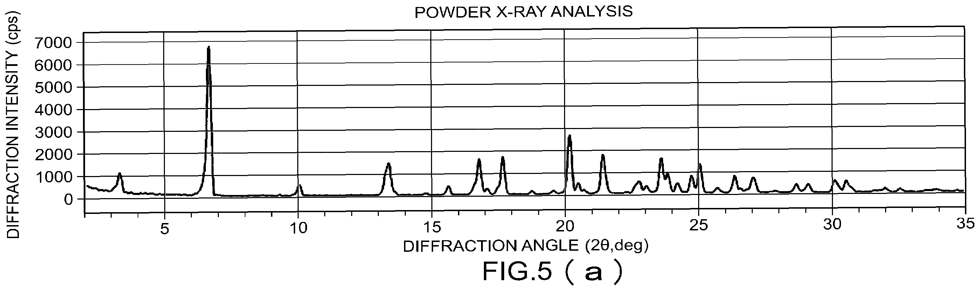

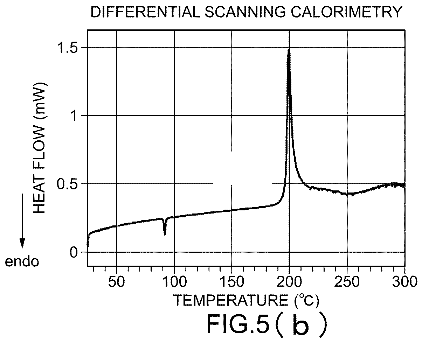

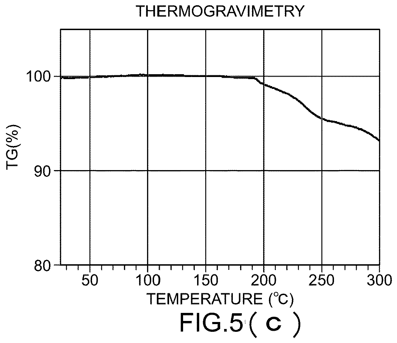

[Fig. 5] An example showing a series of charts of (a) powder X-ray diffraction, (b) differential scanning calorimetry, and (c) thermogravimetry of the crystal of compound A or a derivative thereof (crystal pattern A).

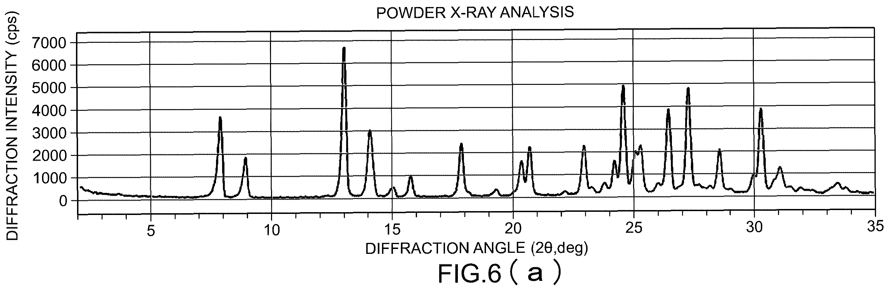

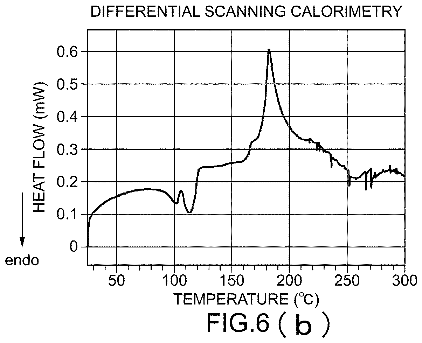

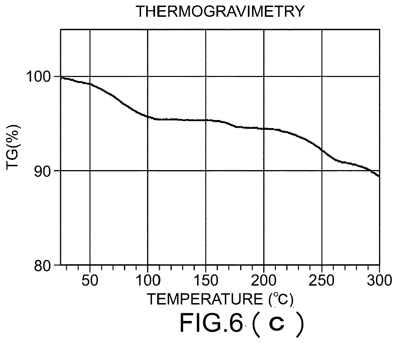

[Fig. 6] An example showing a series of charts of (a) powder X-ray diffraction, (b) differential scanning calorimetry, and (c) thermogravimetry of the crystal of compound A or a derivative thereof (crystal pattern B).

### Description of Embodiments

Hereinafter, a method for predicting the therapeutic response of solid cancer to compound A or a derivative thereof, biomarkers therefor, a pharmaceutical composition containing compound A or a derivative thereof for treating solid cancer characterized by such a biomarker, methods for diagnosing and treating the solid cancer, etc., each included in the aspects of the present invention, will be described in detail.

### [Compound A]

In the scope of the present invention, compound A refers to 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I):

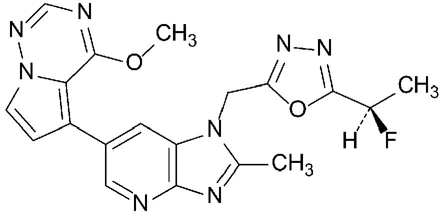

In the scope of the present invention, derivatives of compound A include salts and hydrates of the compound of the formula (I), and optical isomers, tautomers, and solvates of them. The derivative of compound A may be a crystal or a clathrate, and may be in a single crystal form or a mixture of crystal forms, a cocrystal, or the like.

Pharmacologically acceptable salts are preferred as salts of compound A, and examples thereof include salts with an inorganic base, salts with an organic base, salts with an inorganic acid, salts with an organic acid, and salts with a basic or acidic amino acid.

Preferred examples of the salts with an inorganic base include: alkali metal salts such as a sodium salt and a potassium salt; alkaline earth metal salts such as a calcium salt and a magnesium salt; an aluminum salt; and an ammonium salt.

Preferred examples of the salts with an organic base include a salt with any of trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine, and N,N-dibenzylethylenediamine.

Preferred examples of the salts with an inorganic acid include a salt with any of hydrochloric acid, hydrobromic acid, nitric acid, sulfuric acid, and phosphoric acid.

Preferred examples of the salts with an organic acid include a salt with any of formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid.

Preferred examples of the salts with a basic amino acid include a salt with any of arginine, lysine, and ornithine.

Preferred examples of the salts with an acidic amino acid include a salt with any of aspartic acid and glutamic acid.

Details of a method for producing compound A or a derivative thereof are disclosed in International Publication No. WO 2017/188374, and the contents thereof are incorporated herein.

### [Method for Predicting Therapeutic Response of Solid Cancer to

### Compound A]

The method of the present invention for predicting the therapeutic response of solid cancer to compound A or a derivative thereof includes: (a) detecting a genetic character predicted to impart sensitivity to CLK inhibition in a cancer-tissue-derived sample from a subject with solid cancer; and (b) determining an expectation of response to compound A or a derivative thereof in the subject with reference to the genetic character.

The genetic character predicted to impart sensitivity to CLK inhibition includes gene alteration and gene expression alteration. Here, mutation of a gene in the genomic region, for example, mutation such as copy number mutation (CNV), single-nucleotide mutation (SNV), multiple-nucleotide mutation (MNV), insertion or deletion of a small nucleotide sequence (Indel), splicing-site mutation, gene fusion, and translocation of a chromosomal position or site, or RNA splicing alteration may be included in the gene alteration. Examples of the gene can include at least one of ACVR1, AKT1, AKT2, AKT3, ALK, AR, ARAF, BMPR1A, BRAF, CASP8, CCNE1, CDK4, CSF1R, CTNNB1, DDR2, EPAS1, ERBB2, ERBB3, ERBB4, ESR1, EZH2, GNA11, GNAQ, GNAS, H3F3A, HRAS, IDH1, IDH2, JUN, KIT, KLF4, KRAS, MAP2K2, MAP2K4, MAPK1, MDM2, MDM4, MET, MITF, MYCC, MYCN, MYCL, MYOD1, MFE2L2, MKX2-1, NRAS, NTRK1, PDGFRA, PIK3CA, PIK3CB, PLCG1, PTEN, RAC1, RAF1, RET, RHOA, SMO, STAT5B, TBL1XR1, TERT, TP53, TSC2, USP8, WT1, EGFR, FGFR1, FGFR2, FGFR3,MAP2K1,U2AF1, ATR, ATRX, and HIST1H3B.

mRNA level elevation and reduction and protein level elevation and reduction each caused by the gene alteration or not are included in the gene expression alteration.

In an embodiment of the present invention, the genetic character predicted to impart sensitivity to CLK inhibition may be MYC gene amplification, MYC gene mutation, or the transposition of the MYC gene chromosomal position or site, and each of them is detected through comparison with the corresponding reference. Preferably, the subject can be determined as a subject who exhibits particularly high responsivity to compound A or a derivative thereof if a MYC gene copy number is increased as compared with a reference MYC gene copy number, a MYC gene sequence is altered as compared with a reference MYC gene sequence, or a MYC gene chromosomal position or site is translocated as compared with a reference MYC gene chromosomal position or site.

The MYC gene amplification may be one for which increase in copy number as compared with a reference is observed, and may be preferably, but is not limited to, one for which increase in copy number by approximately 1.5 times or more is shown. In another embodiment of the present invention, the MYC gene amplification may be, but is not limited to, one for which increase in copy number by approximately 2 times or more, approximately 4 times or more, approximately 10 times or more, or approximately 20 times or more is shown.

The MYC gene mutation may be mutation of the MYC gene such as single-nucleotide mutation (SNV), multiple-nucleotide mutation (MNV), small insertion or deletion (Indel), splicing-site mutation, and gene fusion of the MYC gene in the genomic region.

The MYC gene mutation may be preferably, but is not limited to, MYCC Q37del, P59L, S146L, MYCN R45Rfs*86, and/or E445K.

In a preferred embodiment of the present invention, the MYC gene can be selected from MYCC, MYCN, and MYCL.

In an embodiment of the present invention, the MYC gene amplification or MYC gene mutation can be detected by a detection technique well known in the art, for example, array comparative genomic hybridization (aCGH), single-nucleotide polymorphism (SNP) arrays, copy number variation (CNV) sequencing, copy number variation (CNV) analysis by real-time PCR or digital PCR, next-generation sequencing (NGS), or multiplex ligation-dependent probe amplification (MLPA).

Likewise, the transposition of the MYC gene chromosomal position or site can be detected by a detection technique well known in the art, for example, in situ hybridization (ISH), fluorescence in situ hybridization (FISH), next-generation sequencing (NGS), or comparative genomic hybridization (CGH). The reference MYC gene copy number, reference MYC gene sequence, and/or reference MYC gene chromosomal position or site may be ones obtained from a noncancer-tissue-derived sample from the same subject, or ones obtained from a database or the like well known in the art such as cBioPortal FOR CANCER GENOMICS.

In an embodiment of the present invention, the genetic character predicted to impart sensitivity to CLK inhibition may be MYC mRNA level elevation or MYC protein level elevation, and this is detected through comparison with a reference. Preferably, the subject can be determined as a subject who exhibits particularly high responsivity to compound A or a derivative thereof if a MYC mRNA level is elevated as compared with a reference MYC mRNA level or a MYC protein level is elevated as compared with a reference MYC protein level.

The MYC mRNA level elevation and/or MYC protein level elevation may be, but is not limited to, one due to MYC gene amplification, MYC gene mutation, and/or the transposition of the MYC gene chromosomal position or site.

The MYC mRNA level elevation may be one for which increase in the mRNA level as compared with a reference is observed, and the MYC protein level elevation may be one for which increase in the protein level as compared with a reference is observed.

The MYC mRNA level can be detected by a detection technique well known in the art, for example, in situ hybridization (ISH), fluorescence in situ hybridization (FISH), next-generation sequencing (NGS), or real-time PCR or digital PCR analysis.

The reference MYC mRNA level may be one obtained from a noncancer-tissue-derived sample from the same subject, or one obtained from a database or the like well known in the art such as cBioPortal FOR CANCER GENOMICS.

The MYC protein level can be detected by a detection technique well known in the art, for example, immunohistochemical staining (IHC), flow cytometry (FACS), enzyme-linked immunosorbent assay (ELISA), proteinarray (Proximity Extension Assay, PEA method), or Western blotting (WB).

The reference MYC protein level may be one obtained from a noncancer-tissue-derived sample from the same subject, or one obtained from a database or the like well known in the art such as cBioPortal FOR CANCER GENOMICS.

In an embodiment of the present invention, the genetic character predicted to impart sensitivity to CLK inhibition is U2AF1 gene mutation, and this is detected through comparison with a reference. Preferably, the subject can be determined as a subject who exhibits particularly high responsivity to compound A or a derivative thereof if a gene sequence of the U2AF1 gene is altered as compared with a reference U2AF1 gene sequence.

The U2AF1 gene mutation may be mutation of the gene such as single-nucleotide mutation (SNV), multiple-nucleotide mutation (MNV), small insertion or deletion (Indel), splicing-site mutation, and gene fusion of the gene in the genomic region, and is preferably S34F mutation or S34Y mutation.

In an embodiment of the present invention, the genetic character predicted to impart sensitivity to CLK inhibition is EGFR gene mutation, POLE gene reduction, CDK12 gene reduction, mutation of a gene selected from one or more of ATRX, ATR, GNAQ, and HIST1H3B, and/or MDM4 exon skip alteration, and each of them is detected through comparison with the corresponding reference. Preferably, the subject can be determined as a subject who exhibits particularly high responsivity to compound A or a derivative thereof if a gene sequence of the EGFR gene is altered as compared with a reference EGFR gene sequence, a POLE gene copy number is decreased as compared with a reference POLE gene copy number, a CDK12 gene copy number is decreased as compared with a reference CDK12 gene copy number, a sequence of a gene selected from one or more of ATRX, ATR, GNAQ, and HIST1H3B is altered as compared with the corresponding reference gene sequence, and/or the MDM4 mRNA sequence is altered as compared with a reference MDM4 mRNA sequence.

The EGFR gene mutation, mutation of a gene selected from one or more of ATRX, ATR, GNAQ, and HIST1H3B, and/or MDM4 exon skip alteration may be each mutation such as single-nucleotide mutation (SNV), multiple-nucleotide mutation (MNV), small insertion or deletion (Indel), splicing-site mutation, and gene fusion, and/or RNA splicing alteration.

The POLE gene reduction or CDK12 gene reduction may be one for which reduction in copy number as compared with the corresponding reference is observed.

The cancer-tissue-derived sample may be a surgical sample, biopsy sample, pleural fluid sample, or ascitic fluid sample obtained from the subject, and may be, for example, a sample collected from one or more of tissues of a lung, blood, a breast, a gastrointestinal tract (a stomach, a colon, a rectum), an ovary, a pancreas, a liver, a bladder, a uterine cervix, a nerve, a uterus, a salivary gland, a kidney, a prostate, a thyroid, and muscle.

The subject is preferably a human subject, but is not limited thereto.

The solid cancer can be selected from one or more of cancers [e.g., large intestine carcinoma (e.g., colon cancer, rectal cancer, anal cancer, familial large intestine carcinoma, hereditary nonpolyposis large intestine carcinoma, gastrointestinal stromal tumor), lung cancer (e.g., non-small-cell lung cancer, small-cell lung cancer, malignant mesothelioma), mesothelioma, pancreatic cancer (e.g., pancreatic ductal carcinoma, pancreatic endocrine tumor), pharyngeal cancer, laryngeal cancer, esophageal carcinoma, gastric cancer (e.g., papillary adenocarcinoma, mucinous adenocarcinoma, adenosquamous cell carcinoma), duodenal cancer, small intestine cancer, breast cancer (e.g., invasive ductal carcinoma, noninvasive ductal carcinoma, inflammatory breast cancer), ovarian cancer (e.g., epithelial ovarian cancer, extragonadal germ cell tumor, ovarian germ cell tumor, ovarian low malignant potential tumor), testicular tumor, prostate cancer (e.g., hormone-dependent prostate cancer, hormoneindependent prostate cancer, castration-resistant prostate cancer), liver cancer (e.g., hepatocellular carcinoma, primary liver cancer, extrahepatic bile duct cancer), thyroid cancer (e.g., medullary thyroid cancer), kidney cancer (e.g., renal cell carcinoma (e.g., clear cell renal cell carcinoma), transitional cell carcinoma in the renal pelvis and ureter), uterine cancer (e.g., cervical cancer, corpus uteri carcinoma, uterine sarcoma), gestational choriocarcinoma, brain tumor (e.g., medulloblastoma, glioma, pineal astrocytic tumor, pilocytic astrocytoma, diffuse astrocytoma, anaplastic astrocytoma, pituitary adenoma), retinoblastoma, skin cancer (e.g., basalioma, malignant melanoma), sarcoma (e.g., rhabdomyosarcoma, leiomyosarcoma, soft part sarcoma, spindle cell sarcoma, clear cell sarcoma), malignant bone tumor, bladder cancer, unknown primary cancer].

In a preferred embodiment of the present invention, the cancer can be selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

In a preferred embodiment of the present invention, the genetic character may be MYCC gene amplification, MYCC gene mutation, or the transposition of the MYCC gene chromosomal position or site in a cancer-tissue-derived sample of ovarian cancer and solid cancer selected from gastric cancer, lung cancer, breast cancer, hepatocellular carcinoma, head and neck squamous cell carcinoma, rectal cancer, bile duct cancer, pancreatic cancer, uveal melanoma, sarcoma, prostate cancer, colon cancer, cutaneous melanoma, mesothelioma, glioblastoma, glioma, cervix squamous cell carcinoma and cervical cancer, testicular germ cell tumor, thymoma, thyroid cancer, renal cell carcinoma, kidney renal papillary cell carcinoma, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a particularly preferred embodiment of the present invention, the genetic character may be MYCC gene amplification, MYCC gene mutation, or the transposition of the MYCC gene chromosomal position or site in a cancer-tissue-derived sample of any of ovarian cancer, breast cancer, gastric cancer, lung cancer, large intestine carcinoma, and prostate cancer.

In a preferred embodiment of the present invention, the genetic character may be MYCN gene amplification, MYCN gene mutation, or the transposition of the MYCN gene chromosomal position or site in a cancer-tissue-derived sample of solid cancer selected from gastric cancer, lung cancer, breast cancer, hepatocellular carcinoma, head and neck squamous cell carcinoma, bile duct cancer, uveal melanoma, sarcoma, prostate cancer, colon cancer, cutaneous melanoma, mesothelioma, glioblastoma, glioma, cervix squamous cell carcinoma and cervical cancer, testicular germ cell tumor, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the genetic character may be MYCN gene amplification, MYCN gene mutation, or the transposition of the MYCN gene chromosomal position or site in a cancer-tissue-derived sample of solid cancer selected from ovarian cancer, breast cancer, gastric cancer, lung cancer, large intestine carcinoma, neuroblastoma, small-cell lung cancer, alveolar rhabdomyosarcoma, medulloblastoma, glioblastoma multiforme, and retinoblastoma.

In a particularly preferred embodiment of the present invention, the genetic character may be MYCN gene amplification, MYCN gene mutation, or the transposition of the MYCN gene chromosomal position or site in a neuroblastoma-tissue-derived sample.

In a preferred embodiment of the present invention, the genetic character may be MYCL gene amplification, MYCL gene mutation, or the transposition of the MYCL gene chromosomal position or site in a cancer-tissue-derived sample of ovarian cancer and solid cancer selected from gastric cancer, lung cancer, breast cancer, hepatocellular carcinoma, head and neck squamous cell carcinoma, rectal cancer, bile duct cancer, uveal melanoma, sarcoma, prostate cancer, colon cancer, cutaneous melanoma, mesothelioma, glioblastoma, glioma, cervix squamous cell carcinoma and cervical cancer, testicular germ cell tumor, thymoma, adrenocortical carcinoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the genetic character may be MYCL gene amplification, MYCL gene mutation, or the transposition of the MYCL gene chromosomal position or site in a cancer-tissue-derived sample of solid cancer selected from ovarian cancer, breast cancer, gastric cancer, lung cancer, large intestine carcinoma, and prostate cancer.

In a further embodiment of the present invention, the method of the present invention may include performing a therapy with compound A or a derivative thereof for a subject in need of treatment of solid cancer according to the prediction result for therapeutic response. The therapy may include administering compound A or a derivative thereof to the subject. The therapy may include not only single administration of compound A or a derivative thereof but also combined administration in combination with another drug and/or combined therapy with another type of therapy.

### [Biomarker]

The biomarker of the present invention is one or more biomarkers selected from the following (a) to (k):
(a) MYC gene amplification;
(b) MYC gene mutation;
(c) MYC gene chromosomal transposition;
(d) MYC mRNA level elevation;
(e) MYC protein level elevation;
(f) EGFR gene mutation;
(g) U2AF1 gene mutation;
(h) POLE gene reduction;
(i) CDK12 gene reduction;
(j) mutation of a gene selected from one or more of ATRX, ATR, GNAQ, and HIST1H3B; and
(k) MDM4 exon skip alteration
for predicting therapeutic response to a therapy with compound A or a derivative thereof in a subject with solid cancer.

The MYC gene amplification, MYC gene mutation, or transposition of the MYC gene chromosomal position or site is detected through comparison with the corresponding reference. Preferably, the subject can be determined as a subject who exhibits particularly high responsivity to compound A or a derivative thereof if a MYC gene copy number is increased as compared with a reference MYC gene copy number, a gene sequence of the MYC gene is altered as compared with a reference MYC gene sequence, or a MYC gene chromosomal position or site is translocated as compared with a reference MYC gene chromosomal position or site.

The MYC gene amplification may be one for which increase in copy number as compared with a reference is observed, and may be preferably, but is not limited to, one for which increase in copy number by approximately 1.5 times or more is shown. In another embodiment of the present invention, the MYC gene amplification may be, but is not limited to, one for which increase in copy number by approximately 2 times or more, approximately 4 times or more, approximately 10 times or more, or approximately 20 times or more is shown.

The MYC gene mutation may be mutation of the MYC gene such as single-nucleotide mutation (SNV), multiple-nucleotide mutation (MNV), small insertion or deletion (Indel), splicing-site mutation, and gene fusion of the MYC gene in the genomic region.

The MYC gene mutation may be preferably, but is not limited to, MYCC Q37del, P59L, S146L, MYCN R45Rfs*86, and/or E445K.

In a preferred embodiment of the present invention, the MYC gene can be selected from the group consisting of MYCC, MYCN, and MYCL.

The MYC mRNA level elevation or MYC protein level elevation is detected through comparison with a reference. Preferably, the subject can be determined as a subject who exhibits particularly high responsivity to compound A or a derivative thereof if a MYC mRNA level is elevated as compared with a reference MYC mRNA level or a MYC protein level is elevated as compared with a reference MYC protein level.

The MYC mRNA level elevation or MYC protein level elevation may be, but is not limited to, one due to MYC gene amplification, MYC gene mutation, or the transposition of the MYC gene chromosomal position or site.

The U2AF1 gene mutation is detected through comparison with a reference. Preferably, the subject can be determined as a subject who exhibits particularly high responsivity to compound A or a derivative thereof if a gene sequence of the U2AF1 gene is altered as compared with a reference U2AF1 gene sequence.

The U2AF1 gene mutation may be mutation of the gene such as single-nucleotide mutation (SNV), multiple-nucleotide mutation (MNV), small insertion or deletion (Indel), splicing-site mutation, and gene fusion of the gene in the genomic region, and is preferably S34F mutation or S34Y mutation.

The EGFR gene mutation, POLE gene reduction, CDK12 gene reduction, mutation of a gene selected from one or more of ATRX, ATR, GNAQ, and HIST1H3B, and/or MDM4 exon skip alteration is detected through comparison with the corresponding reference. Preferably, the subject can be determined as a subject who exhibits particularly high responsivity to compound A or a derivative thereof if a gene sequence of the EGFR gene is altered as compared with a reference EGFR gene sequence, a POLE gene copy number is decreased as compared with a reference POLE gene copy number, a CDK12 gene copy number is decreased as compared with a reference CDK12 gene copy number, a gene sequence of a gene selected from one or more of ATRX, ATR, GNAQ, and HIST1H3B is altered as compared with the corresponding reference gene sequence, and/or the MDM4 mRNA sequence is altered as compared with a reference MDM4 mRNA sequence.

The EGFR gene mutation, mutation of a gene selected from one or more of ATRX, ATR, GNAQ, and HIST1H3B, and/or MDM4 exon skip alteration may be each mutation such as single-nucleotide mutation (SNV), multiple-nucleotide mutation (MNV), small insertion or deletion (Indel), splicing mutation, and gene fusion, and/or RNA splicing alteration.

The POLE gene reduction or CDK12 gene reduction may be one for which reduction in copy number as compared with the corresponding reference is observed.

Each of the references to be used for detection of the biomarkers may be one obtained from a noncancer-tissue-derived sample from the same subject, or one obtained from a database well known in the art. In addition, detection techniques well known in the art can be used, and the detection methods described above can be preferably used, though detecting is not limited thereto.

The subject is preferably a human subject, but is not limited thereto.

The solid cancer can be selected from one or more of cancers [e.g., large intestine carcinoma (e.g., colon cancer, rectal cancer, anal cancer, familial large intestine carcinoma, hereditary nonpolyposis large intestine carcinoma, gastrointestinal stromal tumor), lung cancer (e.g., non-small-cell lung cancer, small-cell lung cancer, malignant mesothelioma), mesothelioma, pancreatic cancer (e.g., pancreatic ductal carcinoma, pancreatic endocrine tumor), pharyngeal cancer, laryngeal cancer, esophageal carcinoma, gastric cancer (e.g., papillary adenocarcinoma, mucinous adenocarcinoma, adenosquamous cell carcinoma), duodenal cancer, small intestine cancer, breast cancer (e.g., invasive ductal carcinoma, noninvasive ductal carcinoma, inflammatory breast cancer), ovarian cancer (e.g., epithelial ovarian cancer, extragonadal germ cell tumor, ovarian germ cell tumor, ovarian low malignant potential tumor), testicular tumor, prostate cancer (e.g., hormone-dependent prostate cancer, hormoneindependent prostate cancer, castration-resistant prostate cancer), liver cancer (e.g., hepatocellular carcinoma, primary liver cancer, extrahepatic bile duct cancer), thyroid cancer (e.g., medullary thyroid cancer), kidney cancer (e.g., renal cell carcinoma (e.g., clear cell renal cell carcinoma), transitional cell carcinoma in the renal pelvis and ureter), uterine cancer (e.g., cervical cancer, corpus uteri carcinoma, uterine sarcoma), gestational choriocarcinoma, brain tumor (e.g., medulloblastoma, glioma, pineal astrocytic tumor, pilocytic astrocytoma, diffuse astrocytoma, anaplastic astrocytoma, pituitary adenoma), retinoblastoma, skin cancer (e.g., basalioma, malignant melanoma), sarcoma (e.g., rhabdomyosarcoma, leiomyosarcoma, soft part sarcoma, spindle cell sarcoma, clear cell sarcoma), malignant bone tumor, bladder cancer, unknown primary cancer].

In a preferred embodiment of the present invention, the solid cancer can be selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

In a preferred embodiment of the present invention, the biomarker of the present invention may be MYCC gene amplification, MYCC gene mutation, or the transposition of the MYCC gene chromosomal position or site as a biomarker for ovarian cancer and solid cancer selected from gastric cancer, lung cancer, breast cancer, hepatocellular carcinoma, head and neck squamous cell carcinoma, rectal cancer, bile duct cancer, pancreatic cancer, uveal melanoma, sarcoma, prostate cancer, colon cancer, cutaneous melanoma, mesothelioma, glioblastoma, glioma, cervix squamous cell carcinoma and cervix adenocarcinoma, testicular germ cell tumor, thymoma, thyroid cancer, renal cell carcinoma, kidney renal papillary cell carcinoma, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a particularly preferred embodiment of the present invention, the biomarker of the present invention may be MYCC gene amplification, MYCC gene mutation, or the transposition of the MYCC gene chromosomal position or site as a biomarker for ovarian cancer, breast cancer, gastric cancer, lung cancer, large intestine carcinoma, and prostate cancer.

In another preferred embodiment of the present invention, the biomarker of the present invention may be MYCN gene amplification, MYCN gene mutation, or the transposition of the MYCN gene chromosomal position or site as a biomarker for solid cancer selected from gastric cancer, lung cancer, breast cancer, hepatocellular carcinoma, head and neck squamous cell carcinoma, bile duct cancer, uveal melanoma, sarcoma, prostate cancer, colon cancer, cutaneous melanoma, mesothelioma, glioblastoma, glioma, cervix squamous cell carcinoma and cervix adenocarcinoma, testicular germ cell tumor, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the biomarker of the present invention may be MYCN gene amplification, MYCN gene mutation, or the transposition of the MYCN gene chromosomal position or site as a biomarker for solid cancer selected from ovarian cancer, breast cancer, gastric cancer, lung cancer, large intestine carcinoma, neuroblastoma, small-cell lung cancer, alveolar rhabdomyosarcoma, medulloblastoma, glioblastoma multiforme, and retinoblastoma.

In a particularly preferred embodiment of the present invention, the biomarker of the present invention may be MYCN gene amplification, MYCN gene mutation, or the transposition of the MYCN gene chromosomal position or site as a biomarker for neuroblastoma.

In another preferred embodiment of the present invention, the biomarker of the present invention may be MYCL gene amplification, MYCL gene mutation, or the transposition of the MYCL gene chromosomal position or site as a biomarker for ovarian cancer and solid cancer selected from gastric adenocarcinoma, lung adenocarcinoma, breast cancer, hepatocellular carcinoma, head and neck squamous cell carcinoma, rectal adenocarcinoma, bile duct cancer, uveal melanoma, sarcoma, prostate adenocarcinoma, colon adenocarcinoma, cutaneous melanoma, mesothelioma, glioblastoma, glioma, cervix squamous cell carcinoma and cervix adenocarcinoma, testicular germ cell tumor, thymoma, adrenocortical carcinoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the biomarker of the present invention may be MYCL gene amplification, MYCL gene mutation, or the transposition of the MYCL gene chromosomal position or site as a biomarker for solid cancer selected from ovarian cancer, breast cancer, gastric cancer, lung cancer, large intestine carcinoma, and prostate cancer.

The biomarker of the present invention can be used as a biomarker for predicting therapeutic response to a therapy with compound A or a derivative thereof.

### [Pharmaceutical Composition]

The pharmaceutical composition of the present invention is a pharmaceutical composition containing compound A or a derivative thereof as an active ingredient for treating solid cancer having a genetic character predicted to impart sensitivity to CLK inhibition.

The genetic character may be selected from one or more of the following (a) to (k):
(a) MYC gene amplification;
(b) MYC gene mutation;
(c) MYC gene chromosomal transposition;
(d) MYC mRNA level elevation;
(e) MYC protein level elevation;
(f) EGFR gene mutation;
(g)U2AF1 mutation;
(h) POLE gene reduction;
(i) CDK12 gene reduction;
(j) mutation of a gene selected from one or more of ATRX, ATR, GNAQ, and HIST1H3B; and
(k) MDM4 exon skip alteration.

The MYC gene amplification, MYC gene mutation, or transposition of the MYC gene chromosomal position or site is detected through comparison with the corresponding reference.

The MYC gene amplification may be one for which increase in copy number as compared with a reference is observed, and may be preferably, but is not limited to, one for which increase in copy number by approximately 1.5 times or more is shown. In another embodiment of the present invention, the MYC gene amplification may be, but is not limited to, one for which increase in copy number by approximately 2 times or more, approximately 4 times or more, approximately 10 times or more, or approximately 20 times or more is shown.

The MYC gene mutation may be mutation of the MYC gene such as single-nucleotide mutation (SNV), multiple-nucleotide mutation (MNV), small insertion or deletion (Indel), splicing-site mutation, and gene fusion of the MYC gene in the genomic region.

The MYC gene mutation may be preferably, but is not limited to, MYCC Q37del, P59L, S146L, MYCN R45Rfs*86, and/or E445K.

In a preferred embodiment of the present invention, the MYC gene can be selected from the group consisting of MYCC, MYCN, and MYCL.

The MYC mRNA level elevation and/or MYC protein level elevation is detected through comparison with a reference.

The MYC mRNA level elevation and/or MYC protein level elevation may be, but is not limited to, one due to MYC gene amplification, MYC gene mutation, or the transposition of the MYC gene chromosomal position or site.

The U2AF1 gene mutation is detected through comparison with a reference.

The U2AF1 gene mutation may be mutation of the gene such as single-nucleotide mutation (SNV), multiple-nucleotide mutation (MNV), small insertion or deletion (Indel), splicing-site mutation, and gene fusion of the gene in the genomic region, and is preferably S34F mutation or S34Y mutation.

The EGFR gene mutation, POLE gene reduction, CDK12 gene reduction, mutation of a gene selected from one or more of ATRX, ATR, GNAQ, and HIST1H3B, and/or MDM4 exon skip alteration is detected through comparison with the corresponding reference.

The EGFR gene mutation, mutation of a gene selected from one or more of ATRX, ATR, GNAQ, and HIST1H3B, and/or MDM4 exon skip alteration may be each mutation such as single-nucleotide mutation (SNV), multiple-nucleotide mutation (MNV), small insertion or deletion (Indel), splicing-site mutation, and gene fusion, and/or RNA splicing alteration.

The POLE gene reduction or CDK12 gene reduction may be one for which reduction in copy number as compared with the corresponding reference is observed.

Each of the references to be used for detection of the genetic character in solid tumor may be one obtained from a noncancer-tissue-derived sample from the same subject, or one obtained from a database well known in the art. In addition, detection techniques well known in the art can be used, and the detection methods described above can be preferably used, though detecting is not limited thereto.

The solid cancer can be selected from one or more of cancers [e.g., large intestine carcinoma (e.g., colon cancer, rectal cancer, anal cancer, familial large intestine carcinoma, hereditary nonpolyposis large intestine carcinoma, gastrointestinal stromal tumor), lung cancer (e.g., non-small-cell lung cancer, small-cell lung cancer, malignant mesothelioma), mesothelioma, pancreatic cancer (e.g., pancreatic ductal carcinoma, pancreatic endocrine tumor), pharyngeal cancer, laryngeal cancer, esophageal carcinoma, gastric cancer (e.g., papillary adenocarcinoma, mucinous adenocarcinoma, adenosquamous cell carcinoma), duodenal cancer, small intestine cancer, breast cancer (e.g., invasive ductal carcinoma, noninvasive ductal carcinoma, inflammatory breast cancer), ovarian cancer (e.g., epithelial ovarian cancer, extragonadal germ cell tumor, ovarian germ cell tumor, ovarian low malignant potential tumor), testicular tumor, prostate cancer (e.g., hormone-dependent prostate cancer, hormoneindependent prostate cancer, castration-resistant prostate cancer), liver cancer (e.g., hepatocellular carcinoma, primary liver cancer, extrahepatic bile duct cancer), thyroid cancer (e.g., medullary thyroid cancer), kidney cancer (e.g., renal cell carcinoma (e.g., clear cell renal cell carcinoma), transitional cell carcinoma in the renal pelvis and ureter), uterine cancer (e.g., cervical cancer, corpus uteri carcinoma, uterine sarcoma), gestational choriocarcinoma, brain tumor (e.g., medulloblastoma, glioma, pineal astrocytic tumor, pilocytic astrocytoma, diffuse astrocytoma, anaplastic astrocytoma, pituitary adenoma), retinoblastoma, skin cancer (e.g., basalioma, malignant melanoma), sarcoma (e.g., rhabdomyosarcoma, leiomyosarcoma, soft part sarcoma, spindle cell sarcoma, clear cell sarcoma), malignant bone tumor, bladder cancer, unknown primary cancer].

In a preferred embodiment of the present invention, the solid cancer can be selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

In a preferred embodiment of the present invention, the genetic character may be MYCC gene amplification, MYCC gene mutation, or the transposition of the MYCC gene chromosomal position or site that is found in ovarian cancer and solid cancer selected from gastric cancer, lung cancer, breast cancer, hepatocellular carcinoma, head and neck squamous cell carcinoma, rectal cancer, bile duct cancer, pancreatic cancer, uveal melanoma, sarcoma, prostate cancer, colon cancer, cutaneous melanoma, mesothelioma, glioblastoma, glioma, cervix squamous cell carcinoma and cervix adenocarcinoma, testicular germ cell tumor, thymoma, thyroid cancer, renal cell carcinoma, kidney renal papillary cell carcinoma, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a particularly preferred embodiment of the present invention, the genetic character may be MYCC gene amplification, MYCC gene mutation, or the transposition of the MYCC gene chromosomal position or site that is found in ovarian cancer, breast cancer, gastric cancer, lung cancer, large intestine carcinoma, and prostate cancer.

In another preferred embodiment of the present invention, the genetic character may be MYCN gene amplification, MYCN gene mutation, or the transposition of the MYCN gene chromosomal position or site that is found in solid cancer selected from gastric cancer, lung cancer, breast cancer, hepatocellular carcinoma, head and neck squamous cell carcinoma, bile duct cancer, uveal melanoma, sarcoma, prostate cancer, colon cancer, cutaneous melanoma, mesothelioma, glioblastoma, glioma, cervix squamous cell carcinoma and cervix adenocarcinoma, testicular germ cell tumor, acute myeloid leukemia, adrenocortical carcinoma, pheochromocytoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the genetic character may be MYCN gene amplification, MYCN gene mutation, or the transposition of the MYCN gene chromosomal position or site that is found in solid cancer selected from ovarian cancer, breast cancer, gastric cancer, lung cancer, large intestine carcinoma, neuroblastoma, small-cell lung cancer, alveolar rhabdomyosarcoma, medulloblastoma, glioblastoma multiforme, and retinoblastoma.

In a particularly preferred embodiment of the present invention, the genetic character may be MYCN gene amplification, MYCN gene mutation, or the transposition of the MYCN gene chromosomal position or site that is found in neuroblastoma.

In another preferred embodiment of the present invention, the genetic character may be MYCL gene amplification, MYCL gene mutation, or the transposition of the MYCL gene chromosomal position or site that is found in ovarian cancer and solid cancer selected from gastric cancer, lung cancer, breast cancer, hepatocellular carcinoma, head and neck squamous cell carcinoma, rectal cancer, bile duct cancer, uveal melanoma, sarcoma, prostate cancer, colon cancer, cutaneous melanoma, mesothelioma, glioblastoma, glioma, cervix squamous cell carcinoma and cervix adenocarcinoma, testicular germ cell tumor, thymoma, adrenocortical carcinoma, esophageal carcinoma, uterine carcinosarcoma, lung squamous cell carcinoma, and bladder urothelial carcinoma (Cell Syst. 2018 Mar 28; 6(3): 282-300.e2).

In a preferred embodiment of the present invention, the genetic character may be MYCL gene amplification, MYCL gene mutation, or the transposition of the MYCL gene chromosomal position or site that is found in solid cancer selected from ovarian cancer, breast cancer, gastric cancer, lung cancer, large intestine carcinoma, and prostate cancer.

The pharmaceutical composition of the present invention can be used for treating solid cancer having the genetic character. The pharmaceutical composition of the present invention can be administered not only through single administration but also in combination with another drug to a subject in need of treatment of the solid cancer. Furthermore, the pharmaceutical composition of the present invention may be one that is used for combined therapy with another type of therapy.

Herein, it should be understood that specific matters described for one embodiment according to an aspect of the present invention may be arbitrarily combined with those for another to form a new embodiment, and such a new embodiment is included in the aspect of the present invention.

### Examples

Hereinafter, the present invention will be more specifically described with examples and others, but the present invention is by no means limited by those examples and others.

### I. Drug Sensitivity Evaluation for Compound A (In Vitro Method)

### Example 1: Evaluation for Different Gene Mutations

The present evaluation was carried out as follows with a conventional procedure of the MANO method described in International Publication No. WO 2018/181863.

### (1) Cell line

HEK (Human Embryonic Kidney) 293 cells and mouse 3T3 fibroblasts were obtained from ATCC (American Type Culture Collection), and maintained with Dulbecco's modified Eagle's medium F-12 (DMEM-F12) supplemented with 10% FBS (both from Thermo Fisher Scientific, Walthman, MA).

### (2) Construction of retroviral vector having random barcode sequence

Into a BamHI restriction enzyme site of a pcx4-bleo vector (https://www.addgene.org/vector-database/2309/), a nucleotide of 60 bp was inserted, and site-directed mutagenesis was then performed with primers each containing a random sequence of 6 bp, producing pcx5-belo with the barcode sequence added thereto. Wild-type cDNA of each of the genes shown in Table 1 below was ligated to the pcx5-belo. In addition, cDNAs encoding mutants were produced with a QuickChange II Site-Directed Mutagenesis Kit (Agilent Technologies, SantaClara, CA), and each of them was ligated to the pcx5-belo.

**[Table 1]**

| | | | | | | |
|---|---|---|---|---|---|---|
| ACVR1 | CCNE1 | EZH2 | KLF4 | MYCN | PTEN | TSC2 |
| AKT1 | CDK4 | GNA11 | KRAS | MYOD1 | RAC1 | USP8 |
| AKT2 | CSF1R | GNAQ | MAP2K2 | NFE2L2 | RAF1 | WT1 |
| AKT3 | CTNNB1 | GNAS | MAP2K4 | NKX2-1 | RET | EGFR |
| ALK | DDR2 | H3F3A | MAPK1 | NRAS | RHOA | FGFR1 |
| AR | EPAS1 | HRAS | MDM2 | NTRK1 | SMO | FGFR2 |
| ARAF | ERBB2 | IDH1 | MDM4 | PDGFRA | STAT5B | FGFR3 |
| BMPR1A | ERBB3 | IDH2 | MET | PIK3CA | TBL1XR1 | MAP2K1 |
| BRAF | ERBB4 | JUN | MITF | PIK3CB | TERT | |
| CASP8 | ESR1 | KIT | MYCC | PLCG1 | TP53 | |

### (3) Preparation of retrovirus and transduction of cell line

The recombinant plasmid prepared as above was introduced into HEK293 cells together with a packaging plasmid (Takara Bio Inc., Shiga, Japan), giving recombinant retroviral particles. In focus forming assay, 3T3 cells were infected with an ecotropic recombinant retrovirus with 4 µg/mL polybrene (Sigma-Aldrich Co. LLC, St. Louis, MO, USA) for 24 hours, and further cultured with DMEM-F12 supplemented with 5% calf serum for up to 2 weeks. The transformation of the cells was evaluated by phase-contrast microscopy or staining with Giemsa solution.

### (4) Detection by MANO method

A region containing the barcode sequence in each cell-lysate-derived genomic DNA was amplified through PCR using primers of 5'-TGCAAAGGACCTTACACAGTCCTG-3' and 5'-GACTCGTTGAAGGGTAGACTAGTC-3'. The conditions for the PCR were as follows.
Initial modification: for 3 minutes at 95°C
Modification: for 15 seconds at 95°C
Annealing: for 30 seconds at 60°C
Elongation: for 60 seconds at 72°C
Number of cycles: 30
Final elongation: for 10 minutes at 72°C

The PCR product after amplification was purified with AMPurebeads (Beckman Coulter, Inc., Brea, CA). A sequencing library was produced with an NEB Next Ultra DNA Library Prep Kit (New England Biolabs, Ipswich, MA) according to the recommendation of the manufacturer. The quality of the library was evaluated with a Qubit 2.0 Fluorometer (Thermo Fisher Scientific) and an Agilent2200 Tape Station system. The library was sequenced by using an Illumina MiSeq (300 cycles) with a Reagent Kit V2 to produce paired-end reads of 150 bp. These sequence reads included the following sequence containing the barcode sequence: 5'-CTAGACTGCCNNNNNNGGATCACTCT-3' (wherein N represents any nucleotide) and the complementary sequence. The amount of each mutant was estimated by counting the number of reads for the barcode sequence. A cell mixture treated with DMSO was used as a reference control for scaling signals of the cell clones. Thus, a signal derived from each treated cell line is calculated as 100 × (median of numbers of reads) / (median of numbers of reads for DMSO control). Each median of numbers of reads is derived from three independent experiments.

### (5) Results

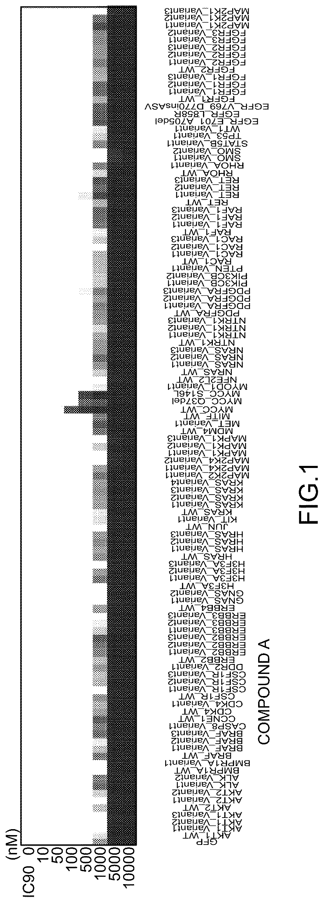

Signals based on the medians of numbers of reads were summarized for every introduced gene (Fig. 1).

Regarding the medians of numbers of reads, a gene group with lower IC90 or IC90 values of compound A than the comparative control GFP or KRASG12D (IC90) is shown in the following Table 2.

**[Table 2]**

| IC90 | GENE | GENE MUTATION/GENE AMPLIFICATION |
|---|---|---|
| | MYCC | WT |
| | MYCC | Q37del |
| | MYCC | S146L |

From those results, compound A was found to exhibit particularly strong growth-inhibitory effect to cells having a mutation of any of those genes.

### Example 2: Evaluation for MYC Gene Mutation

### (1) Cell line

HEK (Human Embryonic Kidney) 293 cells and mouse 3T3 fibroblasts were obtained from ATCC (American Type Culture Collection), and maintained with Dulbecco's modified Eagle's medium F-12 (DMEM-F12) supplemented with 10% FBS (both from Thermo Fisher Scientific, Walthman, MA).

### (2) Construction of retroviral vector having random barcode sequence

The vector used in Example 1 was used.

### (3) Preparation of retrovirus and transduction of cell line

The recombinant plasmid prepared as above was introduced into HEK293 cells together with a packaging plasmid (Takara Bio Inc., Shiga, Japan), giving recombinant retroviral particles. In focus forming assay, 3T3 cells were infected with an ecotropic recombinant retrovirus with 4 µg/mL polybrene (Sigma-Aldrich Co. LLC, St. Louis, MO, USA) for 24 hours, and further cultured with DMEM-F12 supplemented with 5% calf serum for up to 2 weeks. The transformation of the cells was evaluated by phase-contrast microscopy or staining with Giemsa solution.

### (4) Establishment of cell lines

In vitro growth inhibition test was carried out with NIH3T3 cell lines established through gene transfer with individual use of MYCC WT, Q37del, P59L, S146L, MYCN WT, P45Rfs*86, E445K, or EGFR L858R, which had been found in screening, or KRASG12D for comparison.

### (5) Evaluation of cell survival

Each of the cell lines from (4) (1000 cells/90 µL) was seeded in wells of a 96-well plate, and then 10 µL of medium containing DMSO alone or compound A (0.3 nM, 1 nM, 3 nM, 10 nM, 30 nM, 100 nM, 300 nM, 1000 nM, and 3000 nM) was quickly added to give different final concentrations. After culturing for 3 days following the addition, the following evaluation of cell viabilities was carried out.

Evaluation of cell viabilities after different treatments was carried out by measuring intracellular adenosine triphosphate (ATP) with CellTiter-Glo. Specifically, 100 µL of CellTiter-Glo solution was added to each well, incubation was performed at room temperature for 15 minutes, and the chemiluminescence was then measured. The mean of each combination was calculated from the resulting data, and concentration response curves on cell viabilities as the emission intensity in the cells treated with DMSO alone was defined as 100% were drawn for single use or combined use of each agent.

### (6) Results

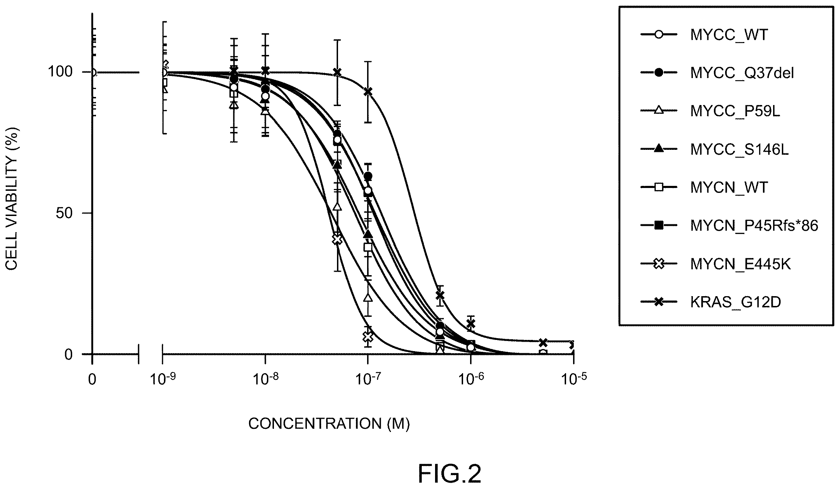

The IC50 values for the NIH3T3 cell lines established through gene transfer with individual use of MYCC WT, Q37del, P59L, S146L, MYCN WT, P45Rfs*86, or E445K were lower than that for the NIH3T3 cell line established through gene transfer with KRASG12D for comparison. It was found from these results that compound A exhibits particularly strong growth-inhibitory effect to cells with overexpression or genetic abnormality of MYCC WT, Q37del, P59L, S146L, MYCN WT, P45Rfs*86, or E445K (Fig. 2).

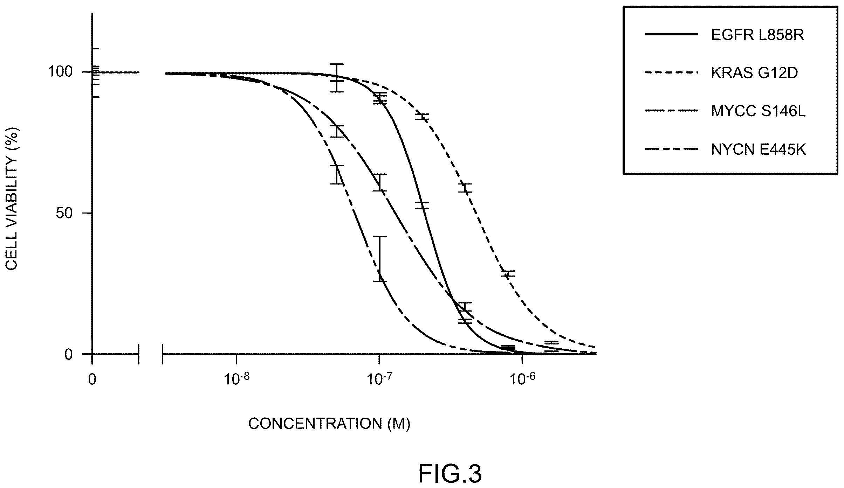

On the other hand, the IC50 values for the NIH3T3 cell lines established through gene transfer with individual use of EGFR L858R, MYCC S146L, or MYCN E445K were lower than that for the NIH3T3 cell line established through gene transfer with KRASG12D for comparison. It was found from these results that compound A exhibits particularly strong growth-inhibitory effect to cells having such gene mutation (Fig. 3).

### Example 3: Evaluation for MYCC gene amplification

### (1) Cell line

An NCI-H82 cell line, AU565 cell line, NCI-H3122 cell line, and PC-9 cell line were obtained from ATCC (American Type Culture Collection), and maintained with Dulbecco's modified Eagle's medium F-12 (DMEM-F12) supplemented with 10% FBS (both from Thermo Fisher Scientific, Walthman, MA).

### (2) MYCC gene amplification in cell lines

According to the database of Cancer Cell Line Encyclopedia, the MYCC gene amplifications of the NCI-H82 cell line and the AU565 cell line were 18.9 and 9.7, respectively. No gene amplification was found in the NCI-H3122 cell line and the PC-9 cell line.

### (3) Evaluation of cell survival

Each of the two cell lines in which MYCC gene amplification was found (1000 cells/90 µL) was seeded in wells of a 96-well plate, and then 10 µL of medium containing DMSO alone or compound A (1 nM, 3 nM, 10 nM, 30 nM, 100 nM, 300 nM, 1000 nM, 3000 nM, and 10000 nM) was quickly added to give different final concentrations. After culturing for 3 days following the addition, the following evaluation of cell viabilities was carried out.

Evaluation of cell viabilities after different treatments was carried out by measuring intracellular adenosine triphosphate (ATP) with CellTiter-Glo. Specifically, 100 µL of CellTiter-Glo solution was added to each well, incubation was performed at room temperature for 15 minutes, and the chemiluminescence was then measured. The mean of each combination was calculated from the resulting data, and concentration response curves on cell viabilities as the emission intensity in the cells treated with DMSO alone was defined as 100% were drawn for single use or combined use of each agent.

### (4) Results

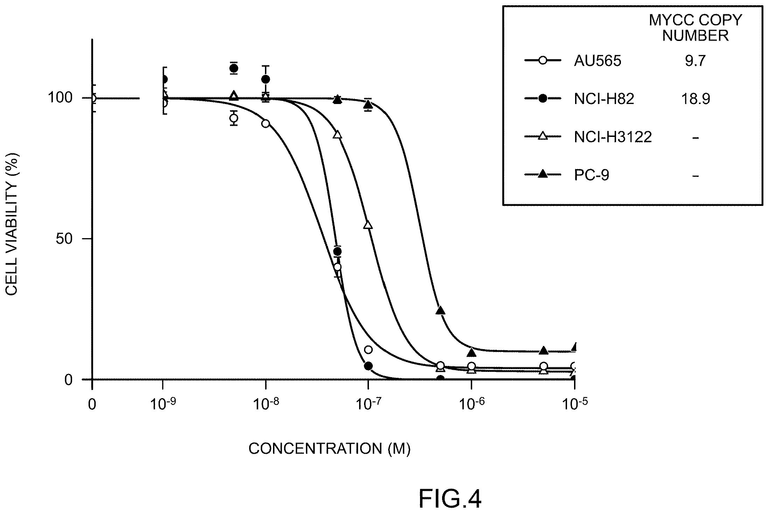

The IC50 values for the NCI-H82 cell line and the AU565 cell line, which had gene amplification of MYCC, were lower than those for the NCI-H3122 cell line and the PC-9 cell line, which did not have such gene amplification. It was found from these results that compound A exhibits particularly high growth-inhibitory effect to cells having overexpression of MYCC (Fig. 4).

### Example 4: Evaluation for MYCN gene amplification

### (1) Culture of cells

The neuroblastoma cell lines CHP-212, SK-N-BE(2), SK-N-DZ, LN-18, U-87MG, and SW1088 were obtained from ATCC (American Type Culture Collection), and cultured with Dulbecco's modified Eagle's medium F-12 (DMEM-F12) supplemented with 10% FBS (both from Thermo Fisher Scientific, Walthman, MA) under conditions of 5% CO₂ and 37°C for use.

### (2) Evaluation of cell survival

Each of the aforementioned six cell lines (4000 cells/90 µL for each case) was seeded in wells of a 96-well plate, and then 10 µL of medium containing DMSO alone or compound A (1 nM, 3 nM, 10 nM, 30 nM, 100 nM, 300 nM, 1000 nM, 3000 nM, and 10000 nM) was quickly added to give different final concentrations. After culturing for 4 days following the addition, the following evaluation of cell viabilities was carried out.

Evaluation of cell viabilities after different treatments was carried out by measuring intracellular adenosine triphosphate (ATP) with CellTiter-Glo. Specifically, 100 µL of CellTiter-Glo solution was added to each well, incubation was performed at room temperature for 15 minutes, and the chemiluminescence was then measured. The mean of each combination was calculated from the resulting data, and concentration response curves on cell viabilities as the emission intensity in the cells treated with DMSO alone was defined as 100% were drawn for single use or combined use of each agent.

### (3) Data analysis

The 50% inhibition concentration (IC50) of compound A was determined by using Graph Pad Prism software version 7.0 (GraphPad Software Inc.).

### (4) Results

The results of evaluation of IC50 for treatment of the different cell lines with compound A are shown in Table 3. The IC50 values for treatment with compound A were lower in all the cell lines in which MYCN gene amplification was found than in the cell lines in which MYCN gene amplification was not found. It was found from these results that compound A exhibits stronger growth-inhibitory effect to cell lines having MYCN gene amplification.

**[Table 3]**

| MYCN GENE AMPLIFICATION AND 50% INHIBITION CONCENTRATION (IC50) IN DIFFERENT NEUROBLASTOMA CELL LINES | | |
|---|---|---|
| CELL LINE | MYCN GENE AMPLIFICATION | IC50 VALUE(nmolZL) |
| CHP-212 | PRESENT | 65.9 |
| SK-N-BE(2) | PRESENT | 86.6 |
| SK-N-DZ | PRESENT | 25.4 |
| LN-18 | ABSENT | 106.3 |
| U-87MG | ABSENT | 101.1 |
| SW1088 | ABSENT | 152.1 |

### II. Evaluation of Antitumor Effect of Compound A to Mouse Subcutaneous Transplantation Models With Different Gene Mutations (In Vivo Method)

### Example 5: MYCC Gene Amplification in Mouse Subcutaneous Transplantation Models With Human-Cancer-Patient-Derived Tumor and Evaluation of Antitumor Effect of Compound A

### (1) Mouse subcutaneous transplantation models

Among mouse subcutaneous transplantation models with different human-cancer-patient-derived tumors, which had been established by National Cancer Center Research Institute, seven models in total with five cancer species of ovarian cancer, lung cancer, gastric cancer, breast cancer, and large intestine cancer, were used for examination in the present test. For these models, results of measurement of MYCC gene amplification have been obtained by National Cancer Center Research Institute.

### (2) Evaluation of antitumor action

A tumor piece in the form of a 2- to 3-mm cube was subcutaneously transplanted into the abdomen of each 6- to 11-week-old female NOG mouse. Calipers were used in measuring tumor volume, which was calculated from the calculation formula: tumor volume = (tumor major axis × tumor minor axis × tumor minor axis) / 2. When the tumor volumes reached approximately 140 to 230 mm³, the mice were grouped into two groups each consisting of two to four mice. Compound A was orally administered in an intermittent manner according to a schedule in which a twice-daily dose of 25 mg/kg was administered twice a week for 14 or 28 days. To a control group, 0.5% methylcellulose solution was orally administered. After the grouping, measurement of tumor volume was performed twice a week. T/C (%) on day 14 or 28 was used as an index of antitumor effect. T/C(%) = (amount of tumor volume change in drug administration group) / (amount of tumor volume change in control group) × 100

### (3) Results

The results of evaluation of the antitumor action of compound A in the mouse subcutaneous transplantation models with human-cancer-patient-derived tumors of five cancer species (ovarian cancer, lung cancer, gastric cancer, breast cancer, and large intestine cancer) are shown in the following Table 4.

**[Table 4]**

| NO | CANCER SPECIES | PDX MODEL NAME | MYCC CNV | T/C(%) |
|---|---|---|---|---|
| 1 | OVARIAN CANCER | NCC_J-PDX_0569TG5 | 8 | -71.1 |
| 2 | GASTRIC CANCER | NCC_J-PDX_0009TG4 | 3 | -44.3 |
| 3 | OVARIAN CANCER | NCC_J-PDX_0735TG5 | 3 | -32.8 |
| 4 | NON-SMALL-CELL LUNG CANCER | NCC_J-PDX_0189TG4 | 4 | -28.0 |
| 5 | BREAST CANCER | NCC_J-PDX_0047TG4 | 2 | -27.8 |
| 6 | LARGE INTESTINE CANCER | NCC_J-PDX_0293TG5 | 2 | -26.1 |
| 7 | NON-SMALL-CELL LUNG CANCER | NCC_J-PDX_0034TG4 | 2 | -9.9 |

| | | | | |
|---|---|---|---|---|
| (IN THE TABLE, THE COLUMN "MYCC CNV" SHOWS MEASUREMENTS OF MYCC GENE AMPLIFICATION.) | | | | |

Compound A exhibited antitumor action with T/C of -71.1%, -44.3%, - 32.8%, and -28.0% (mean: -44.1%) in the models which were used in the present test and had amplification of the MYCC gene (MYCC CNV > 2), and exhibited antitumor action with T/C of -27.8%, -26.1%, and -9.9% (mean: -21.3%) in the models without c-Myc gene amplification (MYCC CNV = 2).

It was found from these results that compound A exhibits stronger antitumor effect to tumors with amplification of the MYCC gene.

Thus, the amplification of the MYCC gene was demonstrated to be useful as a biomarker for predicting the therapeutic response of compound A.

Example 6: Evaluation of Antitumor Effect of Compound A to Mouse Subcutaneous Transplantation Model with Mouse-Derived Tumor Having MMTV-MYCC or MMTV-HRAS

### (1) Evaluation of antitumor action in mouse subcutaneous transplantation model

A tumor piece of the mouse-derived tumor BTG-M605 having MMTV-MYCC or the mouse-derived tumor BTG-R502 having MMTV-HRAS (both from Shanghai Medicilon Inc.) was subcutaneously transplanted into the abdomen of each female nude mouse. Calipers were used in measuring tumor volume, which was calculated from the calculation formula: tumor volume = (tumor major axis × tumor minor axis × tumor minor axis) / 2. When the tumor volumes reached approximately 180 mm³, the mice were grouped into four groups each consisting of eight mice. Compound A was orally administered in an intermittent manner according to a schedule in which a twice-daily dose of 12.5, 25, or 50 mg/kg was administered twice a week (on days 0, 4, 7, and 11) for 14 days. To a control group, 0.5% methylcellulose solution was orally administered. After the grouping, measurement of tumor volume was performed twice a week. T/C (%) on day 14 was used as an index of antitumor effect. T/C(%) = (amount of tumor volume change in drug administration group) / (amount of tumor volume change in control group) × 100

### (2) Results

The results of evaluation of the antitumor action of compound A in the mouse subcutaneous transplantation model with the mouse-derived tumor BTG-M605 having MMTV-MYCC and the mouse subcutaneous transplantation model with the mouse-derived tumor BTG-R502 having MMTV-HRAS are shown in the following Tables 5 and 6, respectively.

**[Table 5]**

| ANTITUMOR EFFECT IN MMTV-MYCC MODEL | | |
|---|---|---|
| GROUP | COMPOUND NAME | T/C(%) |
| 1 | CONTROL GROUP | - |
| 2 | COMPOUND A 12.5 mg/kg | 29.1 |
| 3 | COMPOUND A 25mg/kg | 19.6 |
| 4 | COMPOUND A 50mg/kg | 9.1 |

**[Table 6]**

| ANTITUMOR EFFECT IN MMTV-HRAS MODEL | | |
|---|---|---|
| GROUP | COMPOUND NAME | T/C(%) |
| 1 | CONTROL GROUP | - |
| 2 | COMPOUND A 12.5 mg/kg | 71.1 |
| 3 | COMPOUND A 25mg/kg | 54.4 |
| 4 | COMPOUND A 50mg/kg | 14.4 |

The T/C values of the groups with oral administration of compound A at doses of 12.5, 25, and 50 mg/kg on day 14 were 29.1%, 19.6%, and 9.1%, respectively, for BTG-M605, and 71.1%, 54.4%, and 14.4%, respectively, for BTG-R502, and thus dose-dependent antitumor activity was found in both of the models.

Further comparison of the results for the two models found that compound A exerts stronger antitumor effect to tumor having Myc gene amplification.

Thus, MYCC gene amplification is useful as a biomarker for predicting the therapeutic response of compound A.

Example 7: Evaluation of Antitumor Effect of Compound A to Mouse Subcutaneous Transplantation Model With Tumor Having U2AF1 Mutation From Human Non-Small-Cell Lung Cancer Patient

### (1) Evaluation of Antitumor Action in Mouse Subcutaneous Transplantation Model

LU5231 (Crown Bioscience), a tumor having S34F mutation in the U2AF1 gene from a human non-small-cell lung cancer patient, was suspended in PBS:Cultrex ECM = 1:1, and subcutaneously transplanted into the abdomen of each 6- to 8-week-old female NOD SCID mouse at 1.5 × 10⁵ cells/mouse. Calipers were used in measuring tumor volume, which was calculated from the calculation formula: tumor volume = (tumor major axis × tumor minor axis × tumor minor axis) / 2. When the tumor volumes reached approximately 160 mm³, the mice were grouped into three groups each consisting of nine mice. Compound A was orally administered in an intermittent manner according to a schedule in which a twice-daily dose of 6.25 or 12.5 mg/kg was administered twice a week (days 1, 4, 8, 11, 15, and 18) for 21 days. To a control group, 0.5% methylcellulose solution was orally administered. After the grouping, measurement of tumor volume was performed twice a week. T/C (%) on day 21 was used as an index of antitumor effect.

The human small-cell lung cancer cell line NCI-H1048 (ATCC) without any mutation in the U2AF1 gene was suspended in HBSS:Matrigel = 1:1, and subcutaneously transplanted into the abdomen of each 6-week-old female nude mouse at 4 × 10⁶ cells/mouse. When the tumor volumes reached approximately 150 mm³, the mice were grouped into three groups each consisting of five mice. Compound A was orally administered in an intermittent manner according to a schedule in which a twice-daily dose of 25 or 50 mg/kg was administered twice a week (days 0, 3, 7, and 10) for 14 days. To a control group, 0.5% methylcellulose solution was orally administered. After the grouping, measurement of tumor volume was performed twice a week. T/C (%) on day 14 was used as an index of antitumor effect. T/C(%) = (amount of tumor volume change in drug administration group) / (amount of tumor volume change in control group) × 100

### (2) Results

The results of evaluation of the antitumor action of compound A in LU5231, which was a transplantation model with a tumor having S34F mutation in the U2AF1 gene from a human non-small-cell lung cancer patient, and the mouse subcutaneous transplantation model with the human small-cell lung cancer cell line NCI-H1048 having no S34F mutation in the U2AF1 gene are shown in the following Tables 7 and 8, respectively.

**[Table 7]**

| ANTITUMOR EFFECT IN LU5231 MODEL | | |
|---|---|---|
| GROUP | COMPOUND NAME | T/C(%) |
| 1 | CONTROL GROUP | - |
| 2 | COMPOUND A 6.25mg/kg | 7.0 |
| 3 | COMPOUND A 12.5 mg/kg | -24.2 |

**[Table 8]**

| ANTITUMOR EFFECT IN NCI-H1048 MODEL | | |
|---|---|---|
| GROUP | COMPOUND NAME | T/C(%) |
| 1 | CONTROL GROUP | - |
| 2 | COMPOUND A 25mg/kg | 35.0 |
| 3 | COMPOUND A 50mg/kg | 6.2 |

The T/C values of the groups with oral administration of compound A at doses of 6.25 and 12.5 mg/kg on day 21 in the LU5231 model were 7.0% and -24.2%, respectively, and thus dose-dependent strong antitumor activity with tumor regression was found. On the other hand, the T/C values of the groups with oral administration of compound A at doses of 25 and 50 mg/kg on day 14 in the NCI-H1048 model were 35.0% and 6.2%, respectively.

From these results, compound A was found to exert stronger antitumor effect to tumor having S34F mutation in the U2AF1 gene than to tumor not having the mutation.

Thus, S34F mutation in the U2AF1 gene was demonstrated to be useful as a biomarker for predicting the therapeutic response of compound A.

### III. Mutation Analysis of Cancer-Related Genes

### Example 8: Mutation Analysis of Cancer-Related Genes by Oncomine Comprehensive Assay v3

In the present analysis, as phase I clinical trial of compound A to progressive/recurrent or intractable malignant tumors in accordance with a protocol approved by National Cancer Center Institutional Review Board, tumor tissue specimens in cases with informed consent were obtained and mutation analysis of cancer-related genes by Oncomine Comprehensive Assay v3 was carried out in the following procedure.

### (1) Sequence analysis

Nucleic acid extraction was carried out for the measurement specimens with a RecoverAll (TM) Total Nucleic Acid Isolation Kit for FFPE (Thermo Fisher Scientific). DNA was quantified by fluorometry with a Qubit (TM) 4 Fluorometer (Thermo Fisher Scientific) and a qPCR quantification method with a Step One Plus (TM) Real-Time PCR System (Thermo Fisher Scientific), and RNA was quantified only by fluorometry with a Qubit (TM) 4 Fluorometer, and the nucleic acid concentrations and the amounts of the nucleic acids were determined. From the extracted DNA and RNA, sequence libraries were produced by using Oncomine (TM) Comprehensive Assay v3M (Thermo Fisher Scientific). For reverse transcription in library preparation for RNA, an Ion Torrent (TM) NGS Reverse Transcription Kit (Thermo Fisher Scientific) was used. Sequencing was performed with an Ion Gene Studio (TM) S5 Plus Sequencer (Thermo Fisher Scientific). From data acquired through sequencing, mutations including copy number mutation (CNV), single-nucleotide mutation (SNV), multiple-nucleotide mutation (MNV), insertion/deletion (Indel), and exon skip alteration were identified with an Ion Reporter (TM) Server System (Thermo Fisher Scientific).

### (2) Antitumor effect of compound A

For best overall response in analyzed cases, two cases with partial response (PR) were present for ovarian cancer, and seven cases with stable disease (SD) and five cases with progressive disease (PD) were present for others.

Three of the 14 cases were cases in which the number of days from the day of initial administration to the day of final administration (treatment period) was more than 168 (28 days/cycle × 6 cycles). Two of the three cases were the ovarian cancer cases with PR and the other one case was likewise an ovarian cancer case with SD. On the assumption that strong therapeutic effect was found for the three cases (case A: the best overall response was SD, the treatment period was 186 days, case B: the best overall response was PR, the treatment period was 193 days, case C: the best overall response was PR, the treatment period was 387 days), the correlation with each mutation was analyzed.

### (3) Analysis of correlation with CNV of MYCC, POLE, and CDK12

### (i) CNV of MYCC

In the three ovarian cancer cases in which strong therapeutic effect was found, the CNV of MYCC was 6.49 in case A, 6.24 in case B, and 4.99 in case C, which were equal to or more than the threshold, 3, suggesting the presence of the amplification of CNV in all the three cases. In the others, one transverse colon cancer case (the best overall response was SD, the treatment period was 144 days) exhibited a value of 4.02, and the amplification of CNV was found. On the other hand, there was one ovarian cancer case in which strong therapeutic effect was not found (the best overall response was PD, the treatment period was 11 days), and the CNV of MYCC was 2.58, which was not equal to or more than the threshold, 3. Similarly, the other cases in which strong therapeutic effect was not found did not include a case with the CNV of MYCC being equal to or more than the threshold, 3, which suggested the possibility that the CNV amplification in MYCC allows prediction of the efficacy of compound A. All of those CNV data were such that Filter was PASS or GAIN in the data analysis, and are considered to be data with high reliability.

### (ii) CNV of POLE

In the three ovarian cancer cases in which strong therapeutic effect was found, the upper limit of the 90% confidence interval (95% point) of the CNV of POLE was 0.84 in case C, 1.47 in case A, and 1.94 in case B, which were less than the threshold, 2, suggesting the presence of the reduction of CNV in all the three cases. On the other hand, the upper limit of the 90% confidence interval (95% point) in one ovarian cancer case in which strong therapeutic effect was not found was 3.01 (the best overall response was PD, the treatment period was 11 days), which was not less than the threshold, 2. Similarly, the other 10 cases in which strong therapeutic effect was not found did not include a case with the upper limit of the 90% confidence interval being less than the threshold, 2, which suggested the possibility that the reduction of the CNV of POLE allows prediction of the efficacy of compound A. All of those CNV data were such that Filter was PASS in the data analysis, and are considered to be data with high reliability.

### (iii) CNV of CDK12

In the three ovarian cancer cases in which strong therapeutic effect was found, the upper limit of the 90% confidence interval (95% point) of the CNV of CDK12 was 1.93 in case A, 1.94 in case C, and 1.98 in case B, which were less than the threshold, 2, suggesting the presence of the reduction of CNV in all the three cases. On the other hand, the upper limit of the 90% confidence interval (95% point) in one ovarian cancer case in which strong therapeutic effect was not found was 4.22 (the best overall response was PD, the treatment period was 11 days), which was not less than the threshold, 2. Similarly, the other 10 cases in which strong therapeutic effect was not found did not include a case with the upper limit of the 90% confidence interval being less than the threshold, 2, which suggested the possibility that the reduction of the CNV of CDK12 allows prediction of the efficacy of compound A. All of those CNV data were such that Filter was PASS in the data analysis, and are considered to be data with high reliability.

### (iv) Clinical significance

Those results indicated that the drug efficacy of compound A as a CLK inhibitor can be predicted by confirming the CNV amplification in MYCC, the CNV reduction in POLE, and the CNV reduction in CDK12.

### (4) Analysis of correlation with SNV of ATRX, ATR, GNAQ, and HIST1H3B

### (i) SNV of ATRX, ATR, GNAQ, and HIST1H3B

From SNV, MNV, and Indel in all the cases, mutations considered to have certain qualities (selected with reference to Coverage, %Frequency, and indexes of other qualities, with consideration of the influence of deamination of FFPE for some specimens), to cause the alteration of an amino acid sequence, and not to have high minor allele frequency in East Asians were selected. Among these mutations, mutations present only in ovarian cancer for which strong therapeutic effect was found were p.Val936Leu of ATRX in case C, and p.Thr2487Ala of ATR, p.Ala171Val of GNAQ, and p.Arg84Ser of HIST1H3B in case A. These results suggested the possibility that ATRX, ATR, GNAQ, and/or HIST1H3B possesses functions associated with the efficacy of compound A.

### (ii) Clinical significance

Those results suggested that the deterioration of the normal functions of ATRX, ATR, GNAQ, and/or HIST1H3B because of the mutation of SNV etc., enhances the drug efficacy of compound A as a CLK inhibitor.

### (5) Analysis of correlation with MDM4 exon skip alteration

### (i) MDM4 exon skip alteration

The ratio of the number of sequence reads that allowed detection of connection of Exon5 and Exon7 with Exon6 skipped to the number of sequence reads that allowed detection of connection of Exon1 and Exon2 in the mRNA of MDM4 was high in two PR cases of the three ovarian cancer cases in which strong therapeutic effect was found. The ratio was 0.163 in case C and 0.127 in case B, which were the highest and second highest values among the 14 cases examined.

### (ii) Clinical significance

These results indicated that the drug efficacy of compound A as a CLK inhibitor can be predicted by confirming variants in which Exon6 of MDM4 is skipped.

### Example 9: Reanalysis of CNV by Oncomine Comprehensive Assay v3

In the analysis of CNV in Example 8, the CNV of a tumor cell part in each specimen was estimated with correction considering tumor rates to remove the influence of nontumor cells contaminating specimens. Accordingly, determining tumor rates with high precision is the key to analysis of CNV for clinical tumor specimens. In the present reanalysis, the tumor rates of specimens were obtained through an improved method from the same pathological sections used for obtaining tumor rates in Example 8. In addition, the sequence data acquired in Example 8 were reanalyzed with those tumor rates to newly give desired CNV. The following shows the correlation between CNV given by the present reanalysis and the antitumor effect. In the following, cases A, B, and C are the same as cases A, B, and C in Example 8.

### (1) CNV of MYCC

In the three ovarian cancer cases in which strong therapeutic effect was found, the CNV of MYCC was 6.49 in case A, 7.65 in case B, and 5.41 in case C, which were equal to or more than the threshold, 3, suggesting the presence of the amplification of CNV in all the three cases. In the others, one transverse colon cancer case (the best overall response was SD, the number of days from the day of initial administration to the day of final administration (treatment period) was 144) exhibited a value of 4.02 and one thymoma case (the best overall response was SD, the treatment period was 99 days) exhibited a value of 4.25, and these cases were considered to involve the amplification of CNV because the values were equal to or more than the threshold, 3, although strong therapeutic effect was not found in the cases. On the other hand, there was one ovarian cancer case in which strong therapeutic effect was not found (the best overall response was PD, the treatment period was 11 days), and the CNV of MYCC was 2.58, which was not equal to or more than the threshold, 3. Similarly, the other eight cases in which strong therapeutic effect was not found did not include a case with the CNV of MYCC being equal to or more than the threshold, 3. It was found from those results that a tendency of the amplification of the CNV of MYCC was found to the same degree as or higher degree than in Example 8 for the cases in which strong therapeutic effect was found, suggesting the possibility that the CNV amplification in MYCC allows prediction of the efficacy of compound A. All of those CNV data were such that Filter was PASS or GAIN in the data analysis, and are considered to be data with high reliability.

### (2) CNV of POLE

In the three ovarian cancer cases in which strong therapeutic effect was found, the upper limit of the 90% confidence interval (95% point) of the CNV of POLE was 0.67 in case C, 1.47 in case A, and 1.91 in case B, which were less than the threshold, 2, suggesting the presence of the reduction of CNV in all the three cases. On the other hand, the upper limit of the 90% confidence interval (95% point) in one ovarian cancer case in which strong therapeutic effect was not found was 3.01 (the best overall response was PD, the treatment period was 11 days), which was not less than the threshold, 2. Similarly, the other 10 cases in which strong therapeutic effect was not found did not include a case with the upper limit of the 90% confidence interval being less than the threshold, 2, which suggested the possibility that the reduction of the CNV of POLE allows prediction of the efficacy of compound A. All of those CNV data were such that Filter was PASS in the data analysis, and are considered to be data with high reliability.

### (3) CNV of CDK12

In the three ovarian cancer cases in which strong therapeutic effect was found, the upper limit of the 90% confidence interval (95% point) of the CNV of CDK12 was 1.93 in case A, 1.93 in case C, and 1.97 in case B, which were less than the threshold, 2, suggesting the presence of the reduction of CNV in all the three cases. On the other hand, the upper limit of the 90% confidence interval (95% point) in one ovarian cancer case in which strong therapeutic effect was not found was 4.22 (the best overall response was PD, the treatment period was 11 days), which was not less than the threshold, 2. Similarly, the other 10 cases in which strong therapeutic effect was not found did not include a case with the upper limit of the 90% confidence interval being less than the threshold, 2, which suggested the possibility that the reduction of the CNV of CDK12 allows prediction of the efficacy of compound A. All of those CNV data were such that Filter was PASS in the data analysis, and are considered to be data with high reliability.

### (4) Clinical significance

As with the case of the analysis of CNV in Example 8, those results of the present reanalysis indicated that the drug efficacy of compound A as a CLK inhibitor can be predicted by confirming the CNV amplification in MYCC, the CNV reduction in POLE, and the CNV reduction in CDK12.

### Example 10: Additional Analysis of Mutations of Cancer-Related Genes by Oncomine Comprehensive Assay v3

Mutation analysis of cancer-related genes by Oncomine Comprehensive Assay v3 was carried out for four cases differing from the 14 cases analyzed in Example 8. Also for the four cases, as phase I clinical trial of compound A to progressive/recurrent or intractable malignant tumors in accordance with a protocol approved by National Cancer Center Institutional Review Board, tumor tissue specimens in cases with informed consent were obtained. Sequence analysis was performed in the same manner as in Example 8. However, the calculation method in Example 9 was used in calculating tumor rates for use in analysis of CNV.

### (1) Antitumor effect of compound A

The frequency of administration in two of the four cases subjected to additional analysis was only once and considered insufficient for analyzing the correlation with the antitumor effect of compound A, and hence the residual two cases were analyzed for the correlation with the antitumor effect of compound A.

For best overall response in the two cases, one case with SD and one case with PD were present. The number of days from the day of initial administration to the day of final administration (treatment period) was more than 168 (28 days/cycle × 6 cycles) in one case with SD among those cases. This one case with SD (case D: the best overall response was SD, the treatment period was 386 days), which was a clear cell sarcoma case, was considered to involve strong therapeutic effect, and analyzed for the correlation with each mutation.

### (2) Analysis of correlation with CNV of MYCC, POLE, and CDK12

### (i) CNV of MYCC

In case D, in which strong therapeutic effect was found, the CNV of MYCC was 9.97, which was equal to or more than the threshold, 3, suggesting the presence of the large amplification of CNV. In the other one case, in which strong therapeutic effect was not found, the CNV of MYCC was not equal to or more than the threshold, 3. These results of the present additional analysis also suggested the possibility that the CNV amplification in MYCC allows prediction of the efficacy of compound A. Those CNV data for both the two cases were such that Filter was PASS or GAIN in the data analysis, and are considered to be data with high reliability

### (ii) CNV of POLE

In case D, in which strong therapeutic effect was found, and the other one case, in which strong therapeutic effect was not found, the upper limit of the 90% confidence interval (95% point) of the CNV of POLE was not less than the threshold, 2. This indicated the possibility that compound A exhibits the drug efficacy even if CNV reduction has not occurred in POLE, but was considered not to be a result to deny the possibility that compound A as a CLK inhibitor exhibits the drug efficacy if CNV reduction has occurred in POLE. All of those CNV data were such that Filter was PASS in the data analysis, and are considered to be data with high reliability.

### (iii) CNV of CDK12

In case D, in which strong therapeutic effect was found, and the other one case, in which strong therapeutic effect was not found, the upper limit of the 90% confidence interval (95% point) of the CNV of CDK12 was not less than the threshold, 2. This indicated the possibility that compound A exhibits the drug efficacy even if CNV reduction has not occurred in CDK12, but was considered not to be a result to deny the possibility that compound A as a CLK inhibitor exhibits the drug efficacy if CNV reduction has occurred in CDK12. All of those CNV data were such that Filter was PASS in the data analysis, and are considered to be data with high reliability.

### (iv) Clinical significance

The results of Example 9 and the present additional analysis indicated that the drug efficacy of compound A as a CLK inhibitor can be predicted by confirming the CNV amplification in MYCC, the CNV reduction in POLE, and the CNV reduction in CDK12.

### (3) Analysis of correlation with MDM4 exon skip alteration

### (i) MDM4 exon skip alteration

In case D, in which strong therapeutic effect was found, and the other one case, in which strong therapeutic effect was not found, the ratio of the number of sequence reads that allowed detection of connection of Exon5 and Exon7 with Exon6 skipped to the number of sequence reads that allowed detection of connection of Exon1 and Exon2 in the mRNA of MDM4 was not a large value as in the two cases, shown in Example 8, in which strong therapeutic effect was found. This indicated the possibility that compound A exhibits the drug efficacy without giving a large value, but was considered not to be a result to deny the possibility that compound A exhibits the drug efficacy in the case of a large value.

### (ii) Clinical significance

The results of Example 8 and the present additional analysis indicated that the drug efficacy of compound A as a CLK inhibitor can be predicted by confirming variants in which Exon6 of MDM4 is skipped.

The biomarkers of the present invention are useful as biomarkers for predicting the therapeutic efficacy of a CLK inhibitor such as compound A in treatment of solid cancers including sarcoma.

## Claims

1. A method for predicting therapeutic response to 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 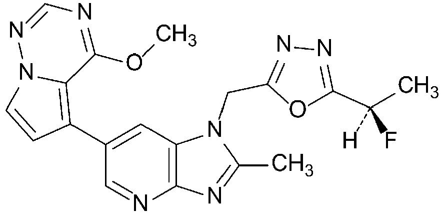 or a derivative thereof in a subject with solid cancer, the method comprising: (1) detecting a genetic character predicted to impart sensitivity to CLK inhibition in a cancer-tissue-derived sample from the subject; and (2) determining an expectation of response to the compound represented by the formula (I) or a derivative thereof in the subject with reference to the genetic character.

2. The method according to claim 1, wherein the genetic character is one or more selected from the group consisting of:
(a) MYC gene amplification;
(b) MYC gene mutation;
(c) MYC gene chromosomal transposition;
(d) MYC mRNA level elevation;
(e) MYC protein level elevation;
(f) EGFR gene mutation;
(g) U2AF1 gene mutation;
(h) POLE gene reduction;
(i) CDK12 gene reduction;
(j) mutation of a gene selected from one or more of ATRX, ATR, GNAQ, and HIST1H3B; and
(k) MDM4 exon skip alteration.

3. The method according to claim 2, comprising:
(1) detecting MYC gene amplification, MYC gene mutation, MYC gene chromosomal transposition, MYC mRNA level elevation, and/or MYC protein level elevation in the cancer-tissue-derived sample from the subject; and
(2) determining that the subject has particularly high responsivity to the compound represented by the formula (I) or a derivative thereof if a MYC gene copy number is increased as compared with a reference MYC gene copy number, a MYC gene sequence is altered as compared with a reference MYC gene sequence, a MYC gene chromosomal position or site is translocated as compared with a reference MYC gene chromosomal position or site, a MYC mRNA level is elevated as compared with a reference MYC mRNA level, and/or a MYC protein level is elevated as compared with a reference MYC protein level.

4. The method according to claim 3, wherein the MYC gene is selected from MYCC, MYCN, and MYCL.

5. The method according to claim 3, wherein the MYC gene copy number is shown to be approximately 1.5 times or more increased as compared with the reference MYC gene copy number, or the MYC gene sequence is shown to be have MYCC Q37del, P59L, S146L, MYCN R45Rfs*86, and/or E445K as compared with a reference.

6. The method according to claim 3, wherein the MYC gene amplification or MYC gene mutation is detected by array comparative genomic hybridization (aCGH), single-nucleotide polymorphism (SNP) arrays, copy number variation (CNV) sequencing, next-generation sequencing (NGS), or multiplex ligation-dependent probe amplification (MLPA).

7. The method according to claim 3, wherein transposition of the MYC gene chromosomal position or site is detected by in situ hybridization (ISH), fluorescence in situ hybridization (FISH), next-generation sequencing (NGS), or comparative genomic hybridization (CGH).

8. The method according to claim 3, wherein the MYC mRNA level elevation is detected by in situ hybridization (ISH), fluorescence in situ hybridization (FISH), next-generation sequencing (NGS), or real-time PCR or digital PCR analysis.

9. The method according to claim 3, wherein the MYC protein level elevation is detected by immunohistochemical staining (IHC), flow cytometry (FACS), enzyme-linked immunosorbent assay (ELISA), proteinarray (Proximity Extension Assay, PEA method), or Western blotting (WB).

10. The method according to claim 2, comprising:
(1) detecting EGFR gene mutation, U2AF1 gene mutation, POLE gene reduction, CDK12 gene reduction, mutation of a gene selected from one or more of ATRX, ATR, GNAQ, and HIST1H3B, and/or MDM4 exon skip alteration in the cancer-tissue-derived sample from the subject; and
(2) determining that the subject has particularly high responsivity to the compound represented by the formula (I) or a derivative thereof if a sequence of a gene selected from one or more of an EGFR gene, a U2AF1 gene, ATRX, ATR, GNAQ, and HIST1H3B is altered as compared with a reference gene sequence for the corresponding gene, and/or an MDM4 mRNA sequence is altered as compared with a reference MDM4 mRNA sequence, and/or a POLE gene copy number or CDK12 gene copy number is decreased as compared with a reference copy number for the corresponding gene.

11. The method according to claim 10, wherein the U2AF1 gene mutation is S34F mutation or S34Y mutation.

12. The method according to claim 10 or 11, wherein the EGFR gene mutation, the U2AF1 gene mutation, the POLE gene reduction, the CDK12 gene reduction, the mutation of a gene selected from one or more of ATRX, ATR, GNAQ, and HIST1H3B, and/or the MDM4 exon skip alteration are/is detected by array comparative genomic hybridization (aCGH), single-nucleotide polymorphism (SNP) arrays, copy number variation (CNV) sequencing, multiplex ligation-dependent probe amplification (MLPA), or RNA sequencing.

13. The method according to any one of claims 1 to 12, wherein the cancer-tissue-derived sample is a surgical sample, biopsy sample, pleural fluid sample, or ascitic fluid sample obtained from the subject, and, for example, is a sample collected from one or more of tissues of a lung, blood, a breast, a gastrointestinal tract (a stomach, a colon, a rectum), an ovary, a pancreas, a liver, a bladder, a uterine cervix, a nerve, a uterus, a salivary gland, a kidney, a prostate, a thyroid, or muscle.

14. The method according to any one of claims 1 to 12, wherein the subject is a human subject.

15. The method according to any one of claims 1 to 12, wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

16. The method according to any one of claims 3 to 9, wherein the MYC gene is MYCC, and the cancer is selected from ovarian cancer, breast cancer, gastric cancer, lung cancer, large intestine carcinoma, and/or prostate cancer.

17. The method according to claim 16, wherein the MYC gene is MYCC, and the cancer is ovarian cancer.

18. The method according to any one of claims 3 to 9, wherein the MYC gene is MYCN, and the cancer is selected from ovarian cancer, breast cancer, gastric cancer, lung cancer, large intestine carcinoma, prostate cancer, neuroblastoma, small-cell lung cancer, alveolar rhabdomyosarcoma, medulloblastoma, glioblastoma multiforme, and/or retinoblastoma.

19. The method according to claim 18, wherein the MYC gene is MYCN, and the cancer is neuroblastoma.

20. The method according to any one of claims 3 to 9, wherein the MYC gene is MYCL, and the cancer is selected from ovarian cancer, breast cancer, gastric cancer, lung cancer, large intestine carcinoma, and/or prostate cancer.

21. A biomarker selected from the following (a) to (k):
(a) MYC gene amplification;
(b) MYC gene mutation;
(c) MYC gene chromosomal transposition;
(d) MYC mRNA level elevation;
(e) MYC protein level elevation;
(f) EGFR gene mutation;
(g) U2AF1 gene mutation;
(h) POLE gene reduction;
(i) CDK12 gene reduction;
(j) mutation of a gene selected from one or more of ATRX, ATR, GNAQ, and HIST1H3B; and
(k) MDM4 exon skip alteration
for predicting therapeutic response to a therapy with 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 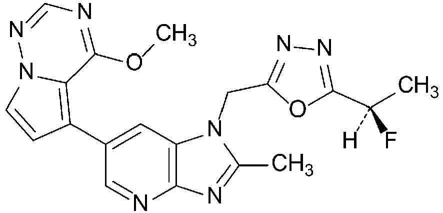 or a derivative thereof in a subject with solid cancer.

22. The biomarker according to claim 21, wherein the MYC gene is selected from MYCC, MYCN, and MYCL.

23. The biomarker according to claim 21, wherein the mutation of the U2AF1 gene is S34F mutation or S34Y mutation.

24. The biomarker according to any one of claims 21 to 23, wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

25. The biomarker according to claim 21, wherein the MYC gene is MYCC, and the cancer is selected from ovarian cancer, breast cancer, gastric cancer, lung cancer, large intestine carcinoma, and prostate cancer.

26. The biomarker according to claim 25, wherein the MYC gene is MYCC, and the cancer is ovarian cancer.

27. The biomarker according to claim 21, wherein the MYC gene is MYCN, and the cancer is selected from ovarian cancer, breast cancer, gastric cancer, lung cancer, large intestine carcinoma, prostate cancer, neuroblastoma, small-cell lung cancer, alveolar rhabdomyosarcoma, medulloblastoma, glioblastoma multiforme, and retinoblastoma.

28. The biomarker according to claim 27, wherein the MYC gene is MYCN, and the cancer is neuroblastoma.

29. The biomarker according to claim 21, wherein the MYC gene is MYCL, and the cancer is selected from ovarian cancer, breast cancer, gastric cancer, lung cancer, large intestine carcinoma, and prostate cancer.

30. A pharmaceutical composition for treating solid cancer having a genetic character predicted to impart sensitivity to CLK inhibition, the pharmaceutical composition comprising 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 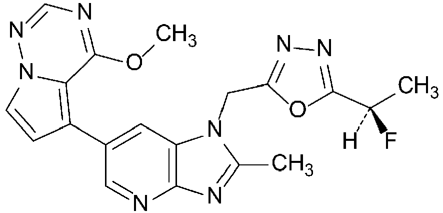 or a derivative thereof as an active ingredient, wherein
the genetic character is one or more selected from the following (a) to (k):
(a) MYC gene amplification;
(b) MYC gene mutation;
(c) MYC gene chromosomal transposition;
(d) MYC mRNA level elevation;
(e) MYC protein level elevation;
(f) EGFR gene mutation;
(g) U2AF1 gene mutation;
(h) POLE gene reduction;
(i) CDK12 gene reduction;
(j) mutation of a gene selected from one or more of ATRX, ATR, GNAQ, and HIST1H3B; and
(k) MDM4 exon skip alteration.

31. The pharmaceutical composition according to claim 30, wherein the MYC gene is selected from MYCC, MYCN, and MYCL.

32. The pharmaceutical composition according to claim 30, wherein the U2AF1 gene mutation is S34F mutation or S34Y mutation.

33. The pharmaceutical composition according to any one of claims 30 to 32, wherein the cancer is selected from one or more of carcinomas, sarcomas including rhabdomyosarcoma such as alveolar rhabdomyosarcoma (including sarcoma derived from a bone, a tendon, cartilage, muscle, fat, a fiber, a blood vessel, adipose, and/or connective tissue), clear cell sarcoma, neuroblastoma, medulloblastoma, astrocytoma, glioblastoma multiforme, retinoblastoma, adenosquamous cell carcinoma, carcinosarcoma, mesodermal mixed tumor, teratocarcinoma, lung cancer (including non-small-cell lung cancer, small-cell lung cancer, adenocarcinoma, and lung squamous cell carcinoma), breast cancer (including metastatic breast cancer), gastrointestinal cancer, gastric cancer, colorectal cancer, colon cancer, rectal cancer, ovarian cancer, pancreatic cancer, liver cancer, bladder cancer, cervical cancer, glioblastoma, uterine cancer, salivary gland cancer, kidney cancer or renal cancer (e.g., Wilms' tumor), prostate cancer, thyroid cancer, and head and neck cancer.

34. The pharmaceutical composition according to claim 30, wherein the MYC gene is MYCC, and the cancer is selected from ovarian cancer, breast cancer, gastric cancer, lung cancer, large intestine carcinoma, and prostate cancer.

35. The pharmaceutical composition according to claim 34, wherein the MYC gene is MYCC, and the cancer is ovarian cancer.

36. The pharmaceutical composition according to claim 30, wherein the MYC gene is MYCN, and the cancer is selected from ovarian cancer, breast cancer, gastric cancer, lung cancer, large intestine carcinoma, neuroblastoma, small-cell lung cancer, alveolar rhabdomyosarcoma, medulloblastoma, glioblastoma multiforme, and retinoblastoma.

37. The pharmaceutical composition according to claim 36, wherein the MYC gene is MYCN, and the cancer is neuroblastoma.

38. The pharmaceutical composition according to claim 30, wherein the MYC gene is MYCL, and selected from ovarian cancer, breast cancer, gastric cancer, lung cancer, large intestine carcinoma, and prostate cancer.
